(19) Europäisches Patentamt
European Patent Office
Office européen des brevets

(11) **EP 2 429 497 B1**

(12) **EUROPEAN PATENT SPECIFICATION**

(45) Date of publication and mention
of the grant of the patent:
**16.08.2017 Bulletin 2017/33**

(51) Int Cl.:
*A61K 9/16* *(2006.01)*        *A61K 9/00* *(2006.01)*
*A61K 31/436* *(2006.01)*

(21) Application number: **10722743.1**

(22) Date of filing: **11.05.2010**

(86) International application number:
**PCT/GB2010/000932**

(87) International publication number:
**WO 2010/130982 (18.11.2010 Gazette 2010/46)**

(54) **PROCESS FOR PREPARING MICROPARTICLES**

VERFAHREN ZUR HERSTELLUNG VON MIKROPARTIKELN

PROCÉDÉ DE PRÉPARATION DE MICROPARTICULES

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **12.05.2009 GB 0908129
12.05.2009 US 216007 P**

(43) Date of publication of application:
**21.03.2012 Bulletin 2012/12**

(73) Proprietor: **Innovata Limited
Chippenham
Wiltshire SN14 6FH (GB)**

(72) Inventors:
• **WHITFIELD, Nicola Kim
Kimberley
Nottingham NG16 2PQ (GB)**
• **MARTYN, Glen
Melton Mowbray
Leicestershire LE13 0HN (GB)**
• **DEY, Fiona
Chippenham
Wiltshire, SN14 6FH (GB)**
• **CREW, Peter
Hamersley
Western Australia 6022 (AU)**

(74) Representative: **Potter Clarkson LLP
The Belgrave Centre
Talbot Street
Nottingham NG1 5GG (GB)**

(56) References cited:
**WO-A1-99/16422        WO-A1-03/043603
WO-A1-2004/030659      WO-A1-2007/086039
WO-A2-02/32406         WO-A2-2005/025535
GB-A- 2 429 646        US-A1- 2004 156 792
US-A1- 2006 134 009**

• **Mahavir Chougule: "Nano-liposomal dry powder
inhaler of tacrolimus: Preparation,
characterization, and pulmonary
pharmacokinetics", Internet , vol. 2, no. 4 1
January 2007 (2007-01-01), pages 675-688,
XP55003572, Retrieved from the Internet:
URL:http://www.ncbi.nlm.nih.gov/pmc/articl
es/PMC2676822/pdf/ijn-2-675.pdf [retrieved on
2011-07-26] cited in the application**

Remarks:
The file contains technical information submitted after
the application was filed and not included in this
specification

**Description**

The invention relates to microparticles, preferably in the form of a dry powder, such as a spray-dried powder, which may be used in medicine, and to a process for forming the microparticles.

Inhalers are widely used to deliver active agents to patients. The active agent may be in the form of a powder. The powders for use in inhalers may be stored and sealed in, for example, blisters or other receptacles, or reservoirs, to provide defined amounts as well as to protect the powders from the environment, in particular moisture.

It is advantageous if the blister or other receptacle, such as a capsule, can be filled by machine rather than hand. However, not all powders have bulk properties which lend themselves to convenient machine filling. For example, powders may be too cohesive or sensitive to moisture. Alternatively, powders may be too light or fine to be easily manipulated.

It is also a requirement that powders for inhalation should be able to be aerosolized from the blister or other receptacle efficiently. This can provide a more efficient dosing regime and avoid waste of expensive active agents.

Excipients have been used to form mixtures with active agents to produce powders which can be suitable for inhalation.

There remains a need for microparticles which can be suitable for machine filling and empty well from receptacles, so as to provide aerosols suitable for inhalation. There also remains a need for microparticles which do not require excipients and complex formulation in order to be suitable for filling and emptying and yet deliver useful aerodynamic properties.

WO 99/32083 describes the use of spray-drying for the production of large, light particles and discloses that microcapsules having properties that are particularly suitable for use in ultrasound diagnostic procedures and for the delivery of a therapeutic agent by inhalation can be prepared by including a blowing agent in the formulation to be spray-dried.

WO 03/080028 discloses a method and apparatus for producing dry particles that contain incompatible components. Ammonium bicarbonate is described as increasing particle porosity.

WO 2007/086039 describes a method of preparing porous microparticles which comprises the steps of combining one or more organic compounds with a volatile system, and drying the system thus formed to provide substantially pure porous microparticles of combinations of organic compounds.

WO 2004/112702 discloses a method of delivering an agent to the pulmonary system of a compromised patient, in a single breath-activated step.

US 6,565,885 describes formulations and methods for the production of perforated microstructures which comprise an active agent.

WO 2005/025540 discloses pharmaceutical compositions which are useful in the treatment of diseases where excess mucus is present in the respiratory tract, such as cystic fibrosis and chronic obstructive pulmonary disease.

Bronchiolitis obliterans (BO) is a fibrotic process resulting in progressive narrowing of bronchiolar lumens and airflow obstruction. The term *obliterative bronchiolitis* (*OB*) is synonymous. Once bronchiolitis obliterans syndrome (BOS) develops, progressive decline in pulmonary function is typical; most patients die of respiratory failure within 5 years of onset. The diagnosis of BOS is usually made by clinical, physiological, and radiographic parameters.

BOS is generally unresponsive, progressive, irreversible, and fatal, with a median survival of approximately 3 years after diagnosis. Therefore, a pharmacologic intervention to prevent the development of BOS and improve survival is urgently needed for the lung transplant population.

Chougule et al (Int. J. Nanomedicine, 2007, 2(4); 675-88) describes a nano-liposomal dry powder inhaler of tacrolimus.

Siswata et al (European Journal of Pharmaceutics and Biopharmaceutics, Volume 69, Issue 3, August 2008, pp 1057-1066) discloses the nebulization of nanoparticulate amorphous or crystalline tacrolimus.

EP 1632208 describes an aerosol preparation comprising an enclosure enclosing an aerosol composition containing a macrolide compound.

WO 2004/041278 discloses a composition comprising specified FK506 derivatives and a β 2-agonist as a combined preparation for treating and preventing acute or chronic asthma.

WO 2005/063242 describes the use of specified macrolide compounds for treating or preventing a pulmonary disease such as airflow obstruction.

WO 97/10806 discloses an aerosol composition comprising a specified tricyclic compound a liquefied hydrofluoroalkane and a medium chain fatty acid triglyceride.

WO 90/14826 describes the use of specified macrolides for the treatment of reversible obstructive airway diseases.

IN 200600953 A discloses aerodynamically light porous dry powder formulations for targeted pulmonary deposition.

WO 2008/127746 describes the enhanced delivery of immunosuppressive drug compositions for pulmonary delivery.

US 6,395,300 discloses porous drug matrices and methods of manufacture thereof.

WO 2004/030659 describes sustained release porous microparticles for inhalation.

The subject matter of the invention is set out in the appended claims.

The term "derivative thereof" preferably refers to a compound which retains the basic skeleton and/or properties of an immunosuppressant but includes one or more substitutions. For example, derivatives may include esters, oximes, carbamates, and pharmaceutically acceptable salts.

As used herein, a "derivative" of tacrolimus preferably means a compound which generally retains the basic skeleton and/or properties of tacrolimus, but includes one or more substitutions.

Other examples of known derivatives include ester and carbamate derivatives at C-32 and/or C-24. Another example are C-22 oxime derivatives. Other examples of suitable known derivatives are disclosed in U.S. Pat. Nos. 5,260,301, 5,196,437, and 5,665,727 the disclosure of which is incorporated herein by reference.

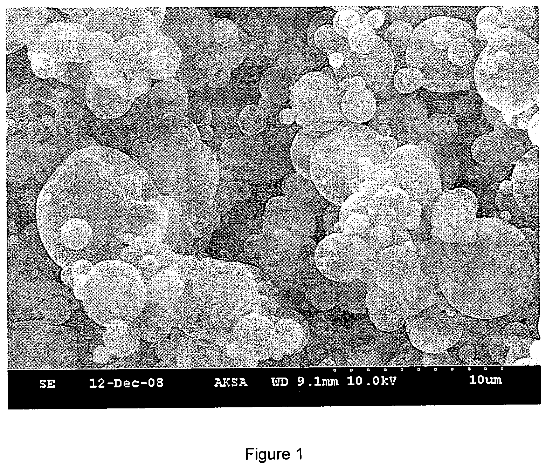

Figure 1 shows scanning electron micrograph pictures of microparticles produced according to the invention.

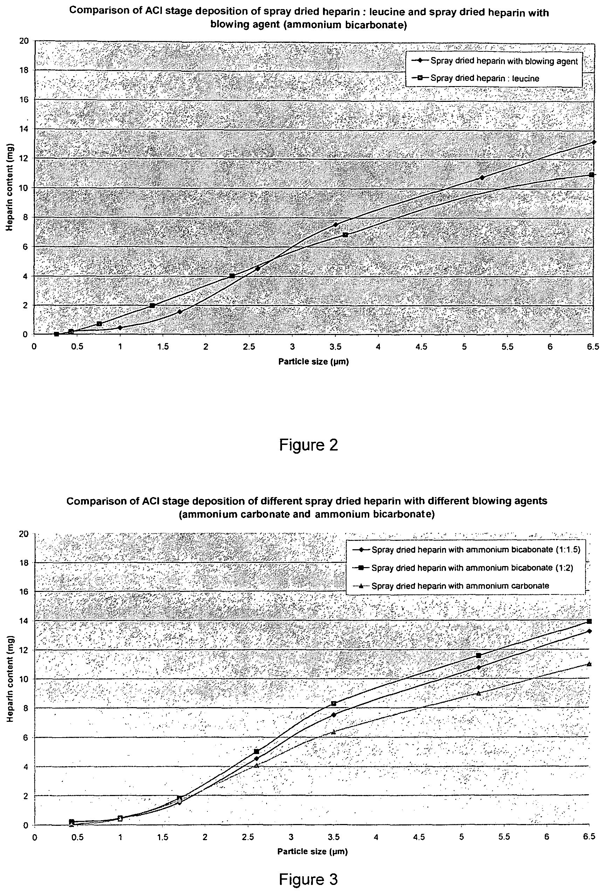

Figure 2 shows a graph of heparin content versus particle size for microparticles produced according to the invention and comparative microparticles .

Figure 3 shows a graph of heparin content versus particle size for microparticles produced according to the invention formed using ammonium bicarbonate and ammonium carbonate.

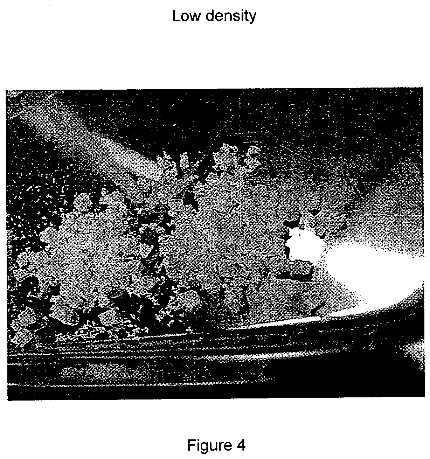

Figure 4 shows a photograph of low density microparticles, in the form of plugs, produced according to the invention.

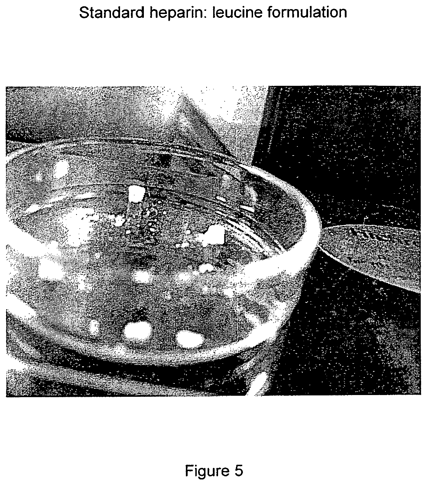

Figure 5 shows a photograph of a standard heparin:leucine formulation.

Figure 6 shows the effect of increasing or decreasing the bicarbonate level on the fine particle mass (FPM) (<5μm).

Figures 7 and 8 show the physical stability and chemical stability of formulations produced according to the invention.

Figures 9 and 10 show SEM images of tacrolimus formulations manufactured with different amounts of ammonium bicarbonate.

Figure 11 shows an SEM image of the formulation containing mannitol as described in Example 11.

In one embodiment, the agent comprises a therapeutic agent, such as a prophylactic agent, or a diagnostic agent. The agent may, for example, be a topical or a systemic drug. Suitable drugs include those for the treatment of asthma, chronic obstructive pulmonary disease (COPD), bronchitis, cystic fibrosis and other lung diseases. A particularly preferred drug comprises heparin or a physiologically acceptable salt thereof, such as heparin sodium.

The agent is typically a saccharide or carbohydrate. By the term "saccharide" or "carbohydrate", it is preferably intended to mean a therapeutic or bioactive agent comprising one or more saccharide or carbohydrate units. The saccharide or carbohydrate units may be naturally occurring or synthetic. The saccharide may or may not comprise non-carbohydrate units in addition to the carbohydrate. The term "therapeutic or bioactive agent" typically does not include components present as carriers, excipients or bulking agents.

In one embodiment, the therapeutic or bioactive agent comprises two or more saccharide or carbohydrate units, such as from about 3 to 100, for example, from 5 to 80 or 10 to 60 units. The units may be the same or different. In one embodiment of the invention, the therapeutic agent comprises an oligosaccharide or a polysaccharide.

In one embodiment, the agent, such as a saccharide (for example heparin or hyaluronic acid), has an average molecular weight greater than about 500 Da or 1,000 Da, such as greater than about 5,000 Da, or greater than about 10,000 Da, for example from about 1,000 to 5,000,000 Da, from about 5,000 to 2,000,000 Da, from about 50,000 to 1,000,000 Da or from about 100,000 to 500,000 Da.

The microparticles typically comprise the agent, such as a saccharide, in an amount of at least about 50 wt.% based on the weight of the microparticles, for example at least about 60 wt.%, such as at least about 70 wt.% or 80 wt.% or 90 wt.%.

In one embodiment of the invention, the microparticles comprise the agent, or saccharide, in an amount of from about 70 to 99 wt.%, from about 80 to 95 wt.%, from about 85 to 90 wt.% or from about 90 wt.% to 95 wt.%.

In one embodiment of the invention, the microparticles consist essentially of the agent, such as a saccharide, and moisture, such as water, and any other components are preferably present in trace amounts, such as less than about 7 wt.%, 2 wt.%, 1 wt.%, 0.1 wt.% or 0.01 wt.%.

Microparticles consisting essentially of an agent may be described as agent microparticles, such as, for example, heparin or heparin sulphate or heparin sodium or heparin sulphate microparticles.

The microparticles, in any of the embodiments defined herein, may comprise one or more of the agents or saccharides described herein. For example, the microparticles of the invention may comprise mixtures of two, three, four, five or more different agents.

In one embodiment, the microparticles comprise, consist essentially of, or consist of, one or more saccharides which are therapeutically active and/or one or more other therapeutic agent as defined herein. For example, the microparticles may comprise, consist essentially, or consist of, a therapeutic glycosaminoglycan, such as heparin or a physiologically acceptable salt thereof. Optionally, the microparticles may also comprise one or more other agents as defined herein. For example, heparin may be used in combination with an antibiotic, such as capreomycin, and/or an antifugal and/or an antiinflammatory agent.

The therapeutically active saccharide, optionally in combination with another agent as defined herein, may be adapted to be a slow release or controlled release formulation.

In one embodiment, the microparticles comprise, comprise, consist essentially of, or consist of, one or more saccharides which are substantially non-therapeutically active or are pharmaceutically acceptable excipients or carriers. For example, the microparticles of the invention may comprise, consist essentially, or consist of, a non-therapeutic glycosaminoglycan, a structural polysaccharide, such as chitin, or pullulan, or monosaccharides, such as glucose, galactose, fructose, xylose and ribose, disaccharides, such as trehalose, maltose, lactose, cellobiose and sucrose, trisaccharides, such as raffinose, acarbose, melezitose or oligosaccharides, such as cyclic oligosaccharides or cyclodextrins.

In an embodiment, the microparticles in which the saccharide is an excipient or carrier comprises one or more therapeutic agents as defined herein. The saccharide preferably comprises a monosaccharide, a disaccharide, preferably trehalose, a trisaccharide or mixtures thereof and/or the therapeutic agent comprises an immunosuppressant. The immunosuppressant may comprise a macrolide such as tacrolimus, sirolimus, pimecrolimus, ciclosporin, everolimus and the like, preferably a calcineurin inhibitor such as tacrolimus. For example, the microparticles of the invention may comprise trehalose and tacrolimus. These microparticles may be suitable for oral or nasal inhalation.

In one embodiment of the invention, the immunosuppressant comprises a compound selected from a macrolide, a calcineurin inhibitor, tacrolimus, sirolimus, pimecrolimus, ciclosporin, everolimus and the like and mixtures thereof, preferably a calcineurin inhibitor such as tacrolimus. In an embodiment of the invention, the immunosuppressant does not comprise a steroid or a corticosteroid, such as prednisone.

In one embodiment of the invention, the therapeutic agent, such as an immunosuppressant, for example tacrolimus, is not liposomally encapsulated or is non-encapsulated (for example, the immunosuppressant comprises at least 70, 80, 90 or 95 wt.% immunosuppressant), although the immunosuppressant may be encapsulated in an embodiment of the invention.

The microparticles which comprise an immunosuppressant, such as tacrolimus, sirolimus, pimecrolimus, ciclosporin, everolimus or the like, or a calcineurin inhibitor, such as tacrolimus, may be used in a chronic immunosuppression inhaled therapy post lung transplant to delay or prevent the onset of acute and/or chronic lung transplant rejection and/or Bronchiolitis Obliterans Syndrome (BOS) and/or BO and increase survival i.e. prophylaxis. The microparticles may be used alone or in combination with other immunosuppressant drugs, such as, for example, steroids, such as, for example, methyl prednisolone/prednisolone and/or an anti-metabolite, such as, for example, azathioprine or mycophenolate mofetil.

Microparticles produced according to the invention comprising an immunosuppressant and an excipient or carrier, are used in the treatment, prevention, mitigation or prophylactic treatment of acute and/or chronic lung transplant rejection and/or BO and/or BOS, by oral and/or nasal inhalation post lung transplant in a subject or mammal i.e. in a subject or mammal which has received a lung transplant. The microparticles may, for example, delay the onset of BO/BOS and/or increase BOS-free survival and/or prevent acute and/or chronic lung transplant rejection. In addition, the microparticles may delay the onset of acute and/or chronic rejection and increase survival.

In one embodiment of the invention, the microparticles comprise one or more immunosuppressants, such as, for example, tacrolimus, sirolimus, pimecrolimus, ciclosporin, everolimus or the like or derivatives or mixtures thereof, and one or more crystalline or non-crystalline excipient or carrier, such as a carrier which is an amorphous or glass form. Such a carrier will preferably exist in the crystalline state such as after spray-drying.

The pharmaceutically acceptable excipient or carrier for use with the immunosuppressant is a saccharide as defined herein. For example, the saccharide may be selected from, for example, monosaccharides, such as glucose, galactose, fructose, xylose and ribose, disaccharides, such as trehalose, maltose, lactose, cellobiose and sucrose, trisaccharides, such as raffinose, acarbose, melezitose or oligosaccharides, such as cyclic oligosaccharides or cyclodextrins or polysaccharides, such as, for example, inulin and maltodextrin. In one embodiment, the saccharide is a disaccharide or higher i.e., a trisaccharide, oligosaccharide or polysaccharide. Alternatively, the saccharide is a disaccharide, such as trehalose.

Suitable crystalline excipient or carrier materials include, for example: monosaccharides such as arabinose, glucose, galactose, galctulose, fructose, mannose, sorbose, xylose, ribulose, ribose, rhamnose; and sugar alcohols such as sorbitol, xylitol, myo-inositol, scyllo-inositol and mannitol. These excipient or carrier materials i.e., monosaccharides such as arabinose, glucose, galactose, galctulose, fructose, mannose, sorbose, xylose, ribulose, ribose, rhamnose; and sugar alcohols such as sorbitol, xylitol, myo-inositol, scyllo-inositol and mannitol, may also be in an amorphous or glass form; these represent embodiments of the invention.

Other useful excipients which may be present in the microparticles or composition and which may exist in the crystalline state after spray-drying but preferably exist in the non-crystalline state such as a glass, include amino acids such as glycine, alanine, serine, cysteine, threonine, valine, proline, methionine, leucine, isoleucine, lysine and arginine, preferably in an amount greater than 50% w/w of the microparticle or powder weight.

In one embodiment of the invention, the excipient or carrier material comprises at least 50 wt.% of the microparticles, such as equal to or greater than 60, 70, 80 or 90 wt.%. For example, the microparticles may comprise from about 40 to about 95 wt.%, or from about 50 to about 90 wt.%, such as from 55 to 85 wt.% excipient or carrier, such as trehalose or mannitol.

In one embodiment of the invention, the excipient or carrier is a hydrophilic material. For example, the excipient or carrier in one embodiment is not a hydrophobic material, such as a surfactant or phospholipid, or a material providing sustained release, such as over 2 hours.

The surface area of the microparticles comprising an immunosuppressant according to the invention may be greater than 1 $m^2$/g and/or less than 5 $m^2$/g as measured by BET in one embodiment of the invention.

In the process of preparing microparticles comprising an immunosuppressant, the feedstock further comprises a blowing material selected from ammonium carbonate or bicarbonate.

Where an excipient or carrier, such as trehalose or mannitol, is included in the solution or dispersion, it is preferred that the blowing material, such as ammonium carbonate or ammonium bicarbonate, is present in an amount of less than 80 wt.% or less than 60 wt.% based on the combined weight of the immunosuppressant and excipient or carrier, such as from 5 to 60 wt.% or from 10 to 30 or 40 wt.%.

In one embodiment, the microparticles comprise an immunosuppressant, such as tacrolimus, in an amount of at least about 40 wt.% based on the weight of the microparticles, for example at least about 50 or 60 wt.%, such as at least about 70 wt.% or 80 wt.%, 90 wt.%, 95, 97, 98 or 99 wt.%.

In one embodiment of the invention, the microparticles comprise the immunosuppressant, such as tacrolimus, in an amount of from about 40 to 99 wt.%, from about 60 to 95 wt.%, from about 70 to 90 wt.% or from about 80 wt.% or 90 wt.% to 95 wt.%. Alternatively, the microparticles may comprise the immunosuppressant in an amount of less than 40 wt.%, such as less than 30 or 20 wt.%, or from 20 to 50 wt.%.

The microparticles or compositions which comprise an immunosuppressant, such as tacrolimus, may also comprise a taste-masking material. Suitable taste masking materials include, for example, aspartame and menthol. The taste masking material is suitably present in an amount to mask the taste of the immunosuppressant.

In one embodiment, the microparticles comprise, consist essentially of, or consist of, one or more saccharides which are therapeutically active and/or one or more other therapeutic agent as defined herein, optionally in combination with one or more saccharides which are substantially non-therapeutically active or are pharmaceutically acceptable excipients. The therapeutically active and non-therapeutically active saccharides may be as defined above.

Examples of agents that can be incorporated within the microparticles include any bioactive substances such as pharmaceutically effective substances, including, but not limited to, anti-inflammatory drugs, analgesics, antiarthritic drugs, antispasmodics, antidepressants, antipsychotics, tranquilizers, antianxiety drugs, narcotic antagonists, antiparkinsonism agents, cholinergic agonists, chemotherapeutic drugs, immunosuppressive agents, antiviral agents, antimicrobial agents, appetite suppressants, anticholinergics, antiemetics, antihistaminics, antimigraine agents, coronary, cerebral or peripheral vasodilators, hormonal agents, contraceptives, antithrombotic agents, diuretics, antihypertensive agents, cardiovascular drugs, opioids, and the like.

In one embodiment of the invention, the agent or microparticles may comprise one or more therapeutic agents selected from 13-cis-retinoic acid, 2-pentenylpenicillin, L-alphaacetylmethadol, S-adenosylmethionine, acebutolol, aceclofenac, acetaminophen, acetaphenazine, acetophenazine, aclidinium, ademetionine, adinazolam, adrafinil, ahnotriptan, albuterol, albuterol, albuterol sulfate, alfentanil, alfentanil HCl, alizapride, allylprodine, alminoprofen, almotriptan, alperopride, alphaprodine, alpidem, alseroxlon, amantadine, ambrisentan, amesergide, amfenac, aminopropylon, amiodarone HCl, amisulpride, amitriptyline, amixetrine, amlodipine, amoxapine, amoxicillin, amperozide, amphenidone, amphetamine, ampicillin, amylpenicillin, andropinirole, anileridine, apazone, apomorphine, apomorphine hydrochloride, apomorphine diacetate, atenolol, atropine sulfate, azacyclonol, azasetron, azatadine, azidocillin, aztreonam, Bacille Calmette-Guerin, baclofen, beclomethasone dipropionate, benactyzine, benmoxine, benoxaprofen, benperidol, benserazide, benzpiperylon, benzquinamide, benztropine, benzydramine, benzylmorphine, benzylpenicillin, bezitramide, binedaline, biperiden, bitolterol, bitolterol mesylate, brofaromine, bromfenac, bromisovalum, bromocriptine, bromopride, bromperidol, brompheniramine, brucine, buclizine, budesonide, formoterol fumarate, budipine, bufexamac, buprenorphine, bupropion, buramate, buspirone, butaclamol, butaperazine, butorphanol, butriptyline, cabergoline, caffeine, calcium-N-carboamoylaspartate, cannabinoids, captodiamine, capuride, carbamazepine, carbcloral, carbenicillin, carbidopa, carbiphene, carbromal, carfecillin, carindacillin, caroxazone, carphenazine, carpipramine, carprofen, cefazolin, cefinetazole, cefmetazole, cefoxitin, ceftazidime, cephacetrile, cephalexin, cephaloglycin, cephaloridine, cephalosporin C, cephalosporins, cephalotin, cephamycin A, cephamycin B, cephamycin C, cephamycins, cepharin, cephradine, cericlamine, cetrizine, chloralbetaine, chlordiazepoxide, chlorobutinpenicillin, chlorpheniramine, chlorpromazine, chlorprothixene, choline, cialis, cilazaprol, cilostazol, cinchophen, cinmetacin, cinnarizine, cipramadol, ciprofloxacin, citalopram, clebopride, clemastine, clobenzepam, clocapramine, clomacran, clometacin, clometocillin, clomipramine, clonidine, clonitazene, clonixin, clopenthixol, clopriac, clospirazine, clothiapine, clovoxamine, cloxacillin, clozapine, codeine, cotinine, cromolyn sodium, cyamemazine, cyclacillin, cyclizine, cyclobenzaprine, cyclosporin A, cyproheptadine, deprenyl, desflurane, desipramine, dexamethasone sodium phosphate, dexfenfluramine, dexmedetomidine, dextroamphetamine, dextromoramide, dextropropoxyphene, diamorphine, diazepam, diclofenac, dicloxacillin, dihydrocodeine, dihydroergokryptine, dihydroergotamine, diltiazem, diphenhydramine, diphenicillin, diphenidol, diphenoxylate, dipipanone, disulfiram, dolasetronmethanesulfonate, domeridone, dornase alfa, dosulepin, doxepin, doxorubicin, doxylamine, dronabinol, droperidol, droprenilamin HCl, duloxetine, eletriptan, eliprodil, enalapril, enciprazine, enflurane, entacapone, entonox, ephedrine, epinephrine, eptastigmine, ergolinepramipexole, ergotamine, ergotamine tartrate, etamiphyllin, etaqualone, ethambutol, ethoheptazine, etodolac, famotidine, fenfluramine, fentanyl, fexofenadine, fibrinogen, fientanyl, flesinoxan, fluconazole, flunisolide, fluoxetine, flupenthixol, fluphenazine, flupirtine, flurazepam, fluspirilene, fluticasone propionate, fluvoxamine, formoterol fumarate, frovatriptan, gabapentin, galanthamine, gepirone, ghrelin, glutathione, glycopyrronium, granisetron, haloperidol, halothane, heliox, heparin, heparin sodium, heparin sulphate, heptylpenicillin, hetacillin, hydromorphone, hydroxyzine, hyoscine, ibuprofen, idazoxan, iloprost, imipramine, indacaterol, indoprofen, insulin (recombinant human), ipratropium bromide, iproniazid, ipsapiraone, isocarboxazid, isoetharine hydrochloride, isoflurane, isometheptene, isoniazid, rifampin, pyrazinamide, ethambutol, icodextrin, isoproterenol, isoproterenol hydrochloride, isoproterenol bitartrate, isosorbide dinitrate, itraconazole, ketamine, ketoprofen, ketorolac, ketotifen, kitanserin, lazabemide, leptin, lesopitron, levalbuterol hydrochloride, levodopa, levofloxacin, levorphanol, lidocaine, lisinopril, lisuride, lofentanil, lofepramine, lomustine, loprazolam, loratidine, lorazepam, lorezepam, loxapine, maprotoline, mazindol, mazipredone, meclofenamate, mecloqualone, medetomidine, medifoxamine, melperone, memantine, menthol, meperidine, meperidine HCl, meptazinol, meropenem, mesoridazine, metampicillin, metaproterenol, metaproterenol sulfate, methacholine chloride, methadone, methaqualone, methicillin, methprylon, methsuximide, methyphenidate, methyprylon, methysergide, metoclopramide, metofenazate, metomidate, metopimazine, metopon, metoprolol, metralindole, mianserin, midazolam, milnacipran, minaprine, mirtazapine, moclobemide; mofegiline, molindrone, mometasone furoate, morphine, nabilone, nadolol, nafcillin, nalbuphine, nalmefene, nalorphine, naloxone, naltrexone, naratriptan, nedocromil, sodium, nefazodone, nefopam, nicergoline, nicotine, nicotine, nifedipine, nisoxetine, nitrous oxide, nitroglycerin, nomifensine, nortriptyline, obestatin, olanzapine, omoconazole, ondansetron, orphenadrine, oxprenolol, oxycodone, palonosetron, papaveretum, papaverine, paroxetine, pemoline, penfluridol, penicillin N, penicillin O, penicillin S, penicillin V, pentamidine isethionate, pentazocine, pentetate, calcium trisodium, pentetate, zinc trisodium, pentobarbital, peptides, pergolike, pericyazine, perphenazine, pethidine, phenazocine, phenelzine, phenobarbital, phentermine, phentolamine, phenyhydrazine, phosphodiesterase-5, pilocarpine, pimozide, pipamerone, piperacetazine, pipotiazine, pirbuterol acetate, pirbuterolnaloxone, piroxicam, pirprofen, pizotifen, pizotyline, polyeptides, polypeptide YY, pramipexole, prentoxapylline, procaine, procaterol HCl, prochlorperazine, procyclidine, promazine, promethazine, propacetamol, propanolol, propentofylline, propofol, propoxyphene, propranolol, proteins, protriptyline, quetiapine, quinine, rasagiline, reboxetine, remacemide, remifentanil, remoxipride, retinol, ribavirin, rimonabant, risperidone, ritanserin, ritodrine, rizatriptan, roxindole, salicylate, salmeterol xinafoate, salmeterol, scopolamine, selegiline, sertindole, sertraline, sevoflurane, sibutramine, sildenafil, spheramine, spiperone, sufentanil, sulpiride, sumatriptan, tacrolimus, tandospirone, terbutaline, terguride, testosterone, testosterone acetate, estosterone enanthate, testosterone proprionate, tetrahydrocannabinol, thioridazine, thiothixene, thrombin, tiagabine, tianeptine, timolol, tiotropium bromide monohydrate, tizanidine, tobramycin, tofenacin, tolcapone, tolfenamate, tolfenamicacid, topiramate, tramadol, tranylcypromine, trazadone, trehalose, triamcinolone acetonide, triethylperazine, trifluoperazine, trifluperidol, triflupromazine, trihexyphenidyl, trimeprazine, trimethobenzamide, trimipramine, tropisetron, tryptophan, vaccine antigens, valproicacid, vardenafil, venlafaxine, verapamil, vigabatrin, viloxazine, yohimbine, zafirlukast, zalospirone, zanamivir, zileuton, ziprasidone, zolmitriptan, zolpidem, zopiclone, zotepine, zuclopenthixol, and salts and combinations thereof.

Suitable agents include therapeutic and prophylactic agents, such as vaccines. These include, but are not limited to, any therapeutically effective biological modifier. Such substances include, but are not limited to, subcellular compositions, cells, bacteria, viruses and molecules including, but not limited to, lipids, organics, proteins and peptides (synthetic and natural), peptide mimetics, hormones (peptide, steroid and corticosteroid), D and L amino acid polymers, oligosaccharides, polysaccharides, nucleotides, oligonucleotides and nucleic acids, including DNA and RNA, protein nucleic acid hybrids, small molecules and physiologically active analogs thereof. Further, the modifiers may be derived from natural sources or made by recombinant or synthetic means and include analogs, agonists and homologs.

As used herein "protein" refers also to peptides and polypeptides. Such proteins include, but are not limited to, enzymes, biopharmaceuticals, growth hormones, growth factors, insulin, antibodies, both monoclonal and polyclonal and fragments thereof, interferons, interleukins and cytokines. Organics include, but are not limited to, pharmaceutically active moieties with aromatic, carbonyl, amino, imino and guanidino groups. Suitable steroid hormones include, but are not limited to, estrogen, progesterone, testosterone and physiologically active analogs thereof. Numerous steroid hormone analogs are known in the art and include, but are not limited to, estradiol, SH-135 and tamoxifen. Many steroid hormones such as progesterone, testosterone and analogs thereof are particularly suitable for use in the present invention. As used herein, "nucleic acids" includes any therapeutically effective nucleic acids known in the art including, but not limited to DNA, RNA and physiologically active analogs thereof. The nucleotides may encode genes or may be any vector known in the art of recombinant DNA including, but not limited to, plasmids, retroviruses and adeno-associated viruses.

Agents which are prophylactically active and carriers therefor are also suitable for the microparticles of the invention. In one embodiment, the microparticles comprise an immunogen such as a vaccine. Suitable vaccines include but are not limited to, live and attenuated viruses, nucleotide vectors encoding antigens, live and attenuated bacteria, antigens, antigens plus adjuvants and haptens coupled to carriers.

In one embodiment of the invention, the agent or saccharide comprises a vaccine in the form of a polysaccharide, or a polysaccharide vaccine.

Typically, polysaccharide vaccines induce antibody production, but do not induce a T-cell response. Suitable polysaccharide vaccines include, for example, vaccines against Meningococcal disease caused by *Neisseria meningitidis* groups A, C, W135 and Y, such as Group C meningococcal vaccine and Menomune®- A/C/Y/W-135, which is a freeze-dried preparation of the group-specific polysaccharide antigens from *Neisseria meningitidis,* Group A, Group C, Group Y and Group W-135. Pneumococcal polysaccharide vaccine (PPV), also known as Pneumovax, is also a suitable vaccine. This is used to prevent *Streptococcus pneumoniae* (pneumococcus) infections such as pneumonia and septicaemia. The vaccine may be a 23-valent vaccine (e.g., Pneumovax II). The Vi capsular polysaccharide vaccine (or ViCPS) is one of two vaccines recommended by the World Health Organisation for the prevention of typhoid (the other is Ty21 a) and is another suitable example.

Other examples include: Typhim Vi®, a Typhoid Vi Polysaccharide Vaccine, produced by Sanofi Pasteur SA, for intramuscular use, which is a sterile solution containing the cell surface Vi polysaccharide extracted from Salmonella enterica serovar Typhi, S typhi Ty2 strain; and Haemophilus b polysaccharide (hem-OFF-fil-us BEE pol-i-SAK-ka-ryd) vaccine which is an active immunizing agent used to prevent infection by *Haemophilus influenzae* type b (Hib) bacteria.

Relatively short chain saccharides can be used in the present invention. In one embodiment, the saccharide comprises a pentasaccharide, such as isolated from an oligosaccharide containing fraction having immunostimulant activity of buffalo milk. A processed oligosaccharide mixture of buffalo milk can induce significant stimulation of antibody, delayed-type hypersensitivity response to sheep red blood cells in BALB/c mice.

In one embodiment of the invention, the agent or saccharide comprises a polysaccharide which is a protein-bound polysaccharide, having antiviral activity, obtained from a marine alga belonging to the genera Nemacystus, Kjellmaniella, Laminaria, Undaria, Hizikia, Porphyra, Gelidium, Gloiopeltis, Gracilaria, Hemineura, Ulva, Spirogyra, Codium and Acetabularia, such as described in US Patent No. 5089481.

In another embodiment, the agent or saccharide comprises a polysaccharide comprising a water-soluble ginseng marc polysaccharide (GMP).

Other examples of suitable polysaccharides include sulphated polysaccharides (SPS). Suitable SPS include those found in marine algae, such as Rhodophyta, Phaeophyta and Chlorophyta, and higher animals. SPS found, for example, in Rhodophyta are galactans consisting entirely of galactose or modified galactose units. They are known commercially as agar and carrageenan. SPS can have anticoagulant activity.

In one embodiment of the invention, the saccharide comprises a glycolipid. Glycolipids are carbohydrate-attached lipids. The head group of a glycolipid is composed of carbohydrates. They occur where a carbohydrate chain is associated with phospholipids on the exoplasmic surface of the cell membrane. Suitable examples include, for example, glyceroglycolipids, galactolipids, sulfolipids (SQDG), glycosphingolipids, cerebrosides, galactocerebrosides, glucocerebrosides glucobicaranateoets, gangliosides (the most complex animal glycolipids; they contain negatively charged oligosaccharides with one or more sialic acid residues; more than 40 different gangliosides have been identified; they are most abundant in cells), globosides, sulfatides, glycophosphosphingolipids (complex glycophospholipids from fungi, including yeasts, and in plants, where they were originally called "phytoglycolipidds", and may comprise as complicated compounds as the negatively charged gangliosides in animals).

In one embodiment, the saccharide is a therapeutic agent comprising an oligosaccharide or polysaccharide which comprises at least two, preferably different, saccharide units. Such therapeutic agents include, for example, streptomycin, glycoproteins and proteoglycans.

Glycoproteins are proteins that contain oligosaccharide chains (glycans) covalently attached to their polypeptide side-chains. Example of glycoproteins include hormones such as, for example, follicle-stimulating hormone, luteinizing hormone, thyroid-stimulating hormone, human chorionic gonadotropin, alpha-fetoprotein and erythropoietin (EPO).

Proteoglycans represent a special class of glycoproteins that are heavily glycosylated. They consist of a core protein with one or more covalently attached glycosaminoglycan (GAG) chain(s). Proteoglycans can be categorised depending upon the nature of their glycosaminoglycan chains. These chains may be: chondroitin sulfate and dermatan sulfate; heparin and heparan sulfate; or keratan sulfate.

Examples of active agents that may be used in the present invention also include cotranscytosis factors, fibrinogen, thrombin, insulin, growth hormone, calcitonin, $\alpha$-antitrypsin, FSH, $\alpha$-interferon, $\beta$-interferon, heparin, Factor VIII, Factor IX, interleukins and blood coagulation factors and mixtures thereof.

In one embodiment of the invention, the agent or saccharide comprises a polysaccharide. The polysaccharide may be a therapeutic polysaccharide. By "therapeutic polysaccharide", it is intended to mean a polysaccharide which can be used in medicine for the prevention, alleviation and/or treatment of one or more diseases.

As used herein, the term "polysaccharide" preferably does not include monosaccharides, such as glucose, galactose, fructose, disaccharides, such as trehalose, maltose, lactose and sucrose, trisaccharides, such as raffinose, acarbose, melezitose or oligosaccharides, such as cyclic oligosaccharides or cyclodextrins and inulin and maltodextrin.

However, in one embodiment of the invention monosaccharides, such as glucose, galactose, fructose, disaccharides, such as trehalose, maltose, lactose and sucrose, trisaccharides, such as raffinose, acarbose, melezitose or oligosaccharides, such as cyclic oligosaccharides or cyclodextrins and inulin and maltodextrin are preferred saccharides for use in the invention. In particular, in one embodiment of the invention, the polysaccharide is inulin.

In one embodiment of the invention, the polysaccharide has an average molecular weight of from about 5,000 to 100,000 Da, from about 7,000 to 50,000 Da, or from about 10,000 to 30,000 Da.

Polysaccharides have a general formula of $C_x(H_2O)_y$ where x is usually a large number between 200 and 2500. Considering that the repeating units in the polymer backbone are often six-carbon monosaccharides, the general formula can also be represented as $(C_6H_{10}O_5)_n$ where n={40...3000}.

Examples of suitable polysaccharides include storage polysaccharides, such as starch and glycogen, structural polysaccharides, such as cellulose and chitin, acidic polysaccharides, bacterial polysaccharides, capsular polysaccharides, xanthan gum, dextran, gellan gum, pullulan, alginate and sodium alginate, and glycan.

Acidic polysaccharides are polysaccharides that contain carboxyl groups, phosphate groups and/or sulfuric ester groups.

Bacterial polysaccharides represent a diverse range of macromolecules that include peptidoglycan, lipopolysaccharides, capsules and exopolysaccharides; compounds whose functions range from structural cell-wall components (eg peptidoglycan), and important virulence factors (eg Poly-N-acetylglucosamine in S. *aureus),* to permitting the bacterium to survive in harsh environments (eg *Pseudomonas aeruginosa* in the human lung).

Pathogenic bacteria commonly produce a thick, mucous-like, layer of polysaccharide. This "capsule" cloaks antigenic proteins on the bacterial surface that would otherwise provoke an immune response and thereby lead to the destruction of the bacteria. Capsular polysaccharides are water soluble, commonly acidic, and have molecular weights in the order of 100-1000 kDa. They are linear and consist of regularly repeating subunits of one to six monosaccharides. There is enormous structural diversity; nearly two hundred different polysaccharides are produced by *E. coli* alone. Mixtures of capsular polysaccharides, either conjugated or native are used as vaccines. Such vaccines can be used in the present invention.

Bacteria and many other microbes, including fungi and algae, often secrete polysaccharides as an evolutionary adaptation to help them adhere to surfaces and to prevent them from drying out. Some of these polysaccharides have been developed into useful products, including xanthan gum, dextran, gellan gum, and pullulan. These may be preferred polysaccharides according to the invention.

The term "glycan" refers to a polysaccharide. Glycan may also be used to refer to the carbohydrate portion of a glycoconjugate, such as a glycoprotein, glycolipid, or a proteoglycan. Glycans usually consist solely of O-glycosidic linkages of monosaccharides. For example, cellulose is a glycan composed of beta-1,4-linked D-glucose, and chitin is a glycan composed of beta-1,4-linked N-acetyl-D-glucosamine. Glycans can be homo or heteropolymers of monosaccharide residues, and can be linear or branched.

In one embodiment of the invention, the agent or saccharide comprises a glycan, such as an O-linked glycoprotein, preferably a glycosaminoglycan or a physiologically acceptable salt or derivative thereof.

Examples of O-linked glycoproteins are: glycophorin, a protein in erythrocyte cell membranes, mucin, a protein in saliva involved in formation of dental plaque and notch, a transmembrane receptor involved in development and cell fate decisions, thrombospondin, Factor VII , Factor IX , and Urinary type Plasminogen Activator.

Glycosaminoglycans are another type of cellular glycan and refer to a group of heteropolysaccharides which contain an N-acetylated hexosamine in a characteristic repeating disaccharide unit. They include polymers such as heparin, heparin sulphate, chondroitin, keratin and dermatan. Due to their polyanionic nature, glycosaminoglycans are "sticky" molecules and they have been found to readily form aggregates when provided in particulate formation. Such aggregates are too large to reach the deep lung in inhalation.

Glycosaminoglycans and their salts or derivatives may be present in a range of molecular weight sizes. Glycosaminoglycans and salts thereof used in the invention suitably have an average molecular weight of from about 8 to 40 kDa, from about 10 to 30 kDa, from about 12 to 20 kDa, such as from about 12 to 18 kDa, 14 to 18 kDa, 15 to 17kDa or 16 to 17kDa. The glycosaminoglycans used in the present invention preferably do not comprise a protein part or core. The number of saccharide units in the polysaccharide chains may be from about 5 to 100, preferably from 10 to 90, more preferably from 20 to 60.

In one embodiment, the glycosaminoglycans used in the present invention will not have been fractionated or fragmented in order to reduce their molecular weight i.e. they will be unfractionated. Unfractionated glycosaminoglycans, such as heparin, include both high and low molecular weight components in a single product. Generally, the glycosaminoglycans will not have been subjected to depolymerization such as by chemical or enzymatic means.

Derivatives of glycosaminoglycans include those glycosaminoglycans subjected to acetylation, deacetylation, oxidation and/or decarboxylation, such as, for example, periodate oxidation. In addition, heparin may be subjected to O-desulphation at, for example, the 2-O and 3-O positions to form suitable derivatives. The glycosaminoglycans used in the present invention can be naturally occurring or synthetic highly sulphated glycosaminoglycans such as, for example, glycosaminoglycan polysulphate compounds or sulfated mucopolysaccharides.

Glycosaminoglycans usually comprise a high degree of negative charge along the polymer from sulphate groups. Physiologically acceptable salts of, for example, glycosaminoglycans, inc lude salts with metallic cations, such as for example, alkali metal, alkaline earth metal or transition metal cations, and ammonium cations. Examples of suitable salts include salts with one or more of lithium, sodium, potassium, calcium, magnesium, zinc and ammonium cations, and mixtures thereof. Sodium salts are preferred.

The glycosaminoglycans may be isolated from natural sources, such as from an animal, or may be synthesised. Commercially available glycosaminoglycans, as well as those described in WO 03/068254 and EP 1511466, may be used in the present invention.

In one embodiment of the invention, the glycosaminoglycan, preferably unfractionated, is selected from one or more of heparin, heparin sulphate, heparitin sulphates, such as heparan sulphate proteoglycan, heparinoids, dermatan, dermatan sulphate, keratin, chondroitin, chondroitin sulphates A, B, C, D and E, heparan, heparan sulphate, keratan sulphate, hyaluronic acid, physiologically acceptable salts thereof, derivatives or fragments thereof, or mixtures of any thereof, preferably the sodium salt of heparin (heparin sodium) or heparin sulfate.

In one embodiment of the invention, the glycosaminoglycan is selected from one or more of heparin, heparin sulphate, chondroitin sulphates A, C, D and E, heparan, heparan sulphate, keratan sulphate, physiologically acceptable salts thereof, derivatives or fragments thereof, or mixtures of any thereof.

In one embodiment of the invention, the glycosaminoglycan is selected from one or more of heparin sodium, heparin sulphate, heparan, heparan sulphate, hyaluronic acid, physiologically acceptable salts thereof, derivatives or fragments thereof, or mixtures of any thereof.

In one embodiment, hyaluronic acid is in the form of a sodium salt. Preferably the sodium salt has a molecular weight of from about 30,000 to 3,000,000 Da.

In another embodiment of the invention, the glycosaminoglycan is selected from the sodium salt of heparin or heparin sulphate, preferably unfractionated heparin or heparin sulphate comprising a mixture of higher and lower molecular weight components.

In one embodiment of the invention, the glycosaminoglycan comprises heparin sodium. Heparin and hyaluronic acid are insoluble in organic solvents.

Heparin is a naturally-occurring polysaccharide which comprises a mixture of variably sulphated polysaccharide chains. Heparin inhibits coagulation, the process whereby thrombosis occurs. Natural heparin consists of molecular chains of varying lengths, or molecular weights. Whole or unfractionated heparin (UFH) may be fractionated to give low and high molecular weight fractions.

Native heparin typically has a molecular weight of from 3 to 50 kDa and this may be used in the present invention. Chains of molecular weight from 5 kDa to over 40 kDa, make up polydisperse pharmaceutical-grade heparin. Commercially available heparin can also have a molecular weight of from 12 to 15 kDa. Both of these may be used in the present invention.

In one embodiment, the heparin comprises UFH i.e. high molecular weight heparin. Alternatively, the heparin used may be a low molecular weight fraction.

In medicine, low molecular weight heparin (LMWH) is a class of medication used as an anticoagulant in diseases that feature thrombosis, as well as for prophylaxis in situations that lead to a high risk of thrombosis.

Heparin derived from natural sources, mainly porcine intestine or bovine lung, can be administered therapeutically to prevent thrombosis. However, the effects of natural, or unfractionated heparin can be difficult to predict.

In one embodiment, the heparin or a physiologically acceptable salt thereof is suitable for inhalation, whereupon it may act, *inter alia,* as a mucolytic.

Low-molecular-weight heparins (LMWHs), in contrast, consist of only short chains of polysaccharide. LMWHs are preferably defined as heparin salts having an average molecular weight of less than 8000 Da and for which at least 60% of all chains have a molecular weight less than 8000 Da. These are obtained by various methods of fractionation or depolymerisation of polymeric heparin. They preferably have a potency of greater than 70 units/mg of anti-factor Xa activity and a ratio of anti-factor Xa activity to anti-thrombin activity of >1.5.

Low molecular weight heparins (LMWHs) and fondaparinux can be used as the saccharide in the present invention. Low molecular weight heparins and fondaparinux can be used to target anti-factor Xa activity rather than anti-thrombin (IIa) activity, with the aim of facilitating a more subtle regulation of coagulation and an improved therapeutic index. Fondaparinux is a synthetic pentasaccharide, whose chemical structure is almost identical to the AT binding pentasaccharide sequence that can be found within polymeric heparin and heparan sulfate.

With LMWH and fondaparinux, there is a reduced risk of osteoporosis and heparin-induced thrombocytopenia (HIT).

Analogues of heparin are commercially available and may also be used in the present invention. Such analogues include sulphated heparin and glycosylated heparin. Heparin derivatives are commonly termed heparinoids and these may also be used in the present invention.

Heparinoids are glycosaminoglycans which are derivatives of heparin. The term has also been used to include naturally occurring and synthetic highly-sulphated polysaccharides of similar structure. Heparinoid preparations have been used for a wide range of applications including as anticoagulants and anti-inflammatories and they have been claimed to have hypolipidemic properties. Heparinoids are preferably mucopolysaccharides obtained from different animal organs, especially from duodenum, through soft extraction processes which guarantee the integrity of the active elements.

Heparinoids include heteropolysaccharides of straight chains with different degrees of sulphation. Examples of heparinoids include danaparoid, danaparoid sodium and a combination of heparin, dermatan sulphate and chondroitin sulphate.

Danaparoid (sodium), a mixture (of the sodium salts) of heparan sulfate, dermatan sulfate, and chondroitin sulfate can be used as an anticoagulant in patients who have developed HIT. Because danaparoid does not contain heparin or heparin fragments, cross-reactivity of danaparoid with heparin-induced antibodies is reported as less than 10%. Other suitable derivatives of heparin include, for example, enoxaparin and dalteparin.

In one embodiment of the invention, the heparin comprises a low molecular weight heparin or a physiologically acceptable salt thereof, such as low molecular weight heparin sodium.

In one embodiment of the invention, the glycosaminoglycan or a physiologically acceptable salt thereof, such as heparin sodium, is used in combination with DNase. The DNase may be any suitable DNase. The DNase may be a DNase I or a DNase II. DNases occur in a number of species and any DNase capable of cleaving DNA may be used. The DNase may be from an animal source, such as bovine or porcine. Generally, however, the DNase is of human origin and is preferably a recombinant DNase. Commercially available DNase preparations such as Dornase™ and Pulmozyme™ may be used. The glycosaminoglycan or DNase may be administered at the same time or one may be administered first, followed by the other.

In a preferred embodiment of the invention, the polysaccharide is not starch, glycogen, cellulose and cellulose derivatives such as methyl cellulose, ethylcellulose and hydroxypropylmethyl cellulose.

By the term "substantially free", it is intended to mean that the excipient or additive is present in an amount of less than about 10 wt.% based on the weight of the microparticles, such as less than about 5 wt.%, less than 2 wt.%, or less than 1 wt.%, such as less than about 0.1 wt.% or 0.01 wt.% or about 0 wt.%.

The microparticles may comprise liquid, such as water or moisture, and/or excipient or carrier material as defined herein and/or taste masking material and/or residual solvent, for example, from the carrier for the solution or dispersion used for atomisation, and/or blowing material, such as volatile solid or decomposition products thereof, in an amount less than about 15 wt.% based on the weight of the microparticles preferably less than about 10 wt.%, such as less than about 5 wt.%, 1 wt.%, 0.5 wt.% or 0.1 wt.%. For example, the microparticles may have a content of liquid, such as water or moisture, and/or an excipient or carrier material as defined herein and/or taste masking material and/or residual solvent, for example, from the carrier for the solution or dispersion used for atomisation, and/or blowing material, such as volatile solid or decomposition products thereof from about 0.1 or 1 to 15 wt.%, from about 0.5 or 5 to 13 wt.%, from about 6 to 12 wt.% or from about 7, 8 or 9 to 10 wt.%. For example, the microparticles may have a content of the above components from about 0.01 to 5 wt.%, from about 0.1 to 4 wt.%, from about 0.5 to 3 wt.% or from about 0.75 to 1, 2 or 2.5 wt.%.

For heparin microparticles, the amount of water is typically from about 8 to 12 wt.%.

In one embodiment the microparticles comprise an immunosuppressant as defined herein, and the content of the above components, such as moisture, may be less than about 5 wt.%, 1 wt.%, 0.5 wt.% or 0.1 wt.%. For example, the microparticles may have a moisture or other component content of from about 0.01 to 5 wt.%, from about 0.1 to 4 wt.%, from about 0.5 to 3 wt.% or from about 0.75 to 1, 2 or 2.5 wt.%.

Tapped bulk density, or tapped or tap density, is the maximum packing density of a powder (or blend of powders) achieved under the influence of well-defined externally applied forces. The minimum packed volume thus achieved depends on a number of factors including particle size distribution, true density, particle shape and cohesiveness due to surface forces including moisture.

The microparticles produced by the invention, comprising a saccharide have a tap density of less than or equal to 0.3 g/cm$^3$, for example less than about 0.23 g/cm$^3$, such as less than about 0.2 g/cm$^3$. For example, in one embodiment, the microparticles invention have a tap density of from about 0.02 to 0.2 g/cm$^3$, from 0.05 to 0.15 g/cm$^3$, or from 0.07 to 0.12 g/cm$^3$, such as from about 0.08 to 0.10 g/ cm$^3$.

In one embodiment of the invention, the microparticles comprising an immunosuppressant have a tap density of from about 0.05 to 0.22 g/ cm$^3$, such as from about 0.1 or 0.15 to 0.2 g/ cm$^3$ or from about 0.12 to 0.18 g/ cm$^3$.

Tap density can be measured by using instruments known to those skilled in the art such as, but not limited to, the Dual Platform Microprocessor Controlled Tap Density Tester (Vankel Technology, Cary, NC) or a GeoPyc™ instrument (Micrometrics Instrument Corp., Norcross, GA 30093). Tap density can be determined using the method of USP Bulk Density and Tapped Density, United States Pharmacopoeia convention, Rockville, MD, 10th Supplement, 4950-4951, 1999. Preferably, the tap density is measured using a Tap Density Volumeter, Copley.

The bulk density of the microparticles may, in any of the embodiments herein, be less than or equal to about 0.25 or 0.2 g/cm$^3$, such as less than about 0.15 g/cm$^3$. In one embodiment, the bulk density is from about 0.02 to 0.15, 0.2 or 0.25 g/cm$^3$, or from about 0.05 to 0.12 g/cm$^3$.

In one embodiment of the invention, the difference between the tap density and the bulk particle density of the microparticles is less than about 0.07 g/cm$^3$ or less than about 0.05 g/cm$^3$ , such as less than 0.03 g/cm$^3$, for example from about 0 to 0.05 g/cm$^3$ or from about 0.01 to 0.03 g/cm$^3$.

In free-flowing powders, the initial bulk and tapped densities will be more similar than in poor flowing powders which yield greater differences between the two values. The term "free-flowing powder" may in one embodiment refer to microparticles where the difference between the tap density and the bulk density is as defined above.

The microparticles comprising an immunosuppressant and a saccharide, have a median geometric diameter (preferably X50 or D50) of less than or equal to about 10 $\mu$m, such as less than about 10 $\mu$m or less than about 5 $\mu$m and, optionally, a tap density of less than or equal to 0.3 g/cm$^3$, such as from for example less than about 0.23 g/cm$^3$, such as less than about 0.2 g/cm$^3$. In one embodiment of the invention, the microparticles have a median geometric diameter (preferably X50 or D50) of from about 1 or 2 to 10 $\mu$m, such as from about 1 to 5 $\mu$m or from 1.5 to 4.5, 1.75 to 4 or 2 to 3 or 3.5 $\mu$m, or from 3.5 to 9 $\mu$m or from about 4 to 8 $\mu$m such as from about 4 to 5, 6 or 7 $\mu$m and a tap density of from about 0.02 to 0.2 g/cm$^3$ , from 0.05 to 0.15 g/cm$^3$, or from 0.07 to 0.12 g/cm$^3$, such as from about 0.08 to 0.10 g/ cm$^3$. The median geometric diameter is measured at a dispersion pressure of 1.0 bar unless stated otherwise.

The median geometric diameter of the microparticles can be measured using a laser diffraction instrument (for example Helos KF, manufactured by Sympatec, Clausthal-Zellerfeld, Germany) as described in Example 1 or using optical techniques (for example using a Morphologi G3 Particle Image Analyser, manufactured by Malvern Instruments Limited, Malvern, UK) as described in Example 7. Other instruments for measuring geometric particle diameter are well known in the art. The diameter of particles in a sample will range depending upon factors such as particle composition and methods of synthesis. The distribution of size of particles in a sample can be selected to permit optimal deposition to targeted sites within the respiratory tract.

For laser diffraction particle sizing systems, the terms "X50" as used herein refers to the median diameter ($\mu$m) as measured on a volume basis, i.e. 50% by volume of the particles are smaller than this diameter and 50% are larger. The term "X90" refers to the median diameter ($\mu$m) measured on a volume basis wherein 90% of the particles are smaller than this diameter and 10% are larger. The term "X10" refers to the median diameter ($\mu$m) measured on a volume basis wherein 10% of the particles are smaller than this diameter and 90% are larger. Laser diffraction measuring systems include, as an example, Sympatec HELOS system or Malvern Mastersizer 2000.

For optical particle sizing systems, the terms "D50" as used herein refers to the median diameter ($\mu$m) as measured on a number basis by a laser diffraction particle sizing system, i.e. 50% by number of the particles are smaller than this diameter and 50% are larger. The term "D90" refers to the median diameter ($\mu$m) measured on a number basis wherein 90% of the particles are smaller than this diameter and 10% are larger. The term "D10" refers to the median diameter ($\mu$m) measured on a number basis wherein 10% of the particles are smaller than this diameter and 90% are larger. Optical measuring systems include, as an example, Malvern Morphologi G3 Particle Image Analyser.

In one embodiment of the invention, a reference to "median diameter" or "median geometric diameter" in any embodiment herein is a reference to the X50 or D50.

The term "mass median aerodynamic diameter" or "MMAD" is defined as the median of the distribution of mass with respect to aerodynamic diameter. The median aerodynamic diameter and the geometric standard deviation are used to describe the particle size distribution of an aerosol, based on the mass and size of the particles. The median (50%) particle size is obtained from a linear regression analysis of the cumulative distribution data. According to such a description, fifty percent of the particles by mass will be smaller than the median aerodynamic diameter, and fifty percent of the particles will be larger than the median aerodynamic diameter.

A common technique or apparatus for measuring the mass median aerodynamic diameter (MMAD) of a powder for inhalation is the Andersen Cascade Impactor (ACI). The aerodynamic particle size distribution and/or MMAD of the powder may also be determined using a Next Generation Impactor (NGI).

The microparticles according to the invention in any embodiment typically have a mass median aerodynamic diameter (MMAD) of equal to or less than about 10 μm, such as from about 0.1 to 10 μm.

In one embodiment, the MMAD is from about 1 μm to about 5 or 6 μm. In another embodiment of the invention, the MMAD is from about 1 μm to about 3 μm. In a further embodiment, the MMAD is from about 2, 3 or 4 μm to about 5 or 6 μm such as from 2 to 4 or 2 to 3 μm. The microparticles may, for example, be microparticles comprising an immunosuppressant and, optionally, an excipient as defined herein.

The particles may be for localized delivery to selected regions of the respiratory tract such as the deep lung or upper or central airways. Particles having an MMAD ranging from about 3 to about 5 μm are preferred for delivery to the central and upper airways. Particles having an MMAD ranging from about 1 to about 3 μm are preferred for delivery to the deep lung. In one embodiment, microparticles administered to the respiratory tract travel through the upper airways (oropharynx and larynx), the lower airways which include the trachea followed by bifurcations into the bronchi and bronchioli and through the terminal bronchioli which in turn divide into respiratory bronchioli leading then to the ultimate respiratory zone, the alveoli or the deep lung. The microparticles may impact at any stage.

The Carr's Index is based on the decrease in powder volume during tapping and can be used to predict flowability (R.L. Carr, (1965), Chem Eng. 72, 163-168). The lower the number, the more free-flowing the powder. An increase in the value is proportional to adhesion and friction properties of a powder, including (attractive) triboelectric charge.

Carr's Index (or Carr's Compressibility Index), C, can be calculated using the following formulae:

$$C = 100 \times (V_o - V_f)/V_o \text{ or } 100 \times (D_o - D_f)/D_o$$

where "V" and "D" represent powder volume and density respectively, subscript "o" denotes the initial or untapped state and "f" the final or tapped state, or as described in Example 2.

The microparticles or powder according to the invention preferably have a Carr's Index of less than about 30%, such as less than about 26%, 25% or 23%, or from about 5% to 30%, from about 10% to 23% or 26%, or from about 15 or 19 to 23 or 26% or from about 20 to 26% and optionally the microparticles have a median geometric diameter (preferably X50 or D50) of from about 1 or 2 to 10 μm, from about 2 or 3.5 to 9 μm, or from about 3 or 4 to 8 μm, such as from about 1 to 5 μm, 1.5 to 4.5, 1.75 to 4 or 2 to 3 or 3.5 μm, 2 to 2.7 μm or from 4 to 5, 6, 7 or 8 μm.

In one embodiment of the invention, the microparticles comprising an immunosuppressant have a Carr's Index of less than about 30%, such as less than about 26%, 25% or 23%. For example, the microparticles may have a Carr's Index of from about 20 or 21 to 29%, 22 or 23 to 27 or 24 or 25 to 26%.

In one aspect of the invention, microparticles or a powder are provided which have a Carr's Index of from about 5% to 30%, from about 10% to 25%, from about 20 to 26 % or from about 15 or 19 to 23% and optionally the microparticles have a median geometric diameter (preferably X50 or D50) of from about 1 or 2 to 10 μm, from about 3.5 to 9 μm, or from about 4 to 7 μm, such as from about 1 to 5 μm, 1.5 to 4.5, 1.75 to 4 or 2 to 3 or 3.5 μm or about 4 to 5, 6, 7 or 8 μm.

In one embodiment of the invention, the microparticles comprise a saccharide as defined above and have a tap density less than or equal to about 0.2 g/cm$^3$ and a median geometric diameter (preferably X50 or D50) of from about 3.5 to 10 μm, optionally a Carr's Index less than 25%, such as from about 18 to 24%, and optionally an MMAD from about 0.1 to 10 μm.

In a preferred embodiment of the invention, the microparticles comprise a saccharide as defined above and have a tap density from about 0.15 to 0.2 g/cm$^3$ and a median geometric diameter (preferably X50 or D50) of less than about 10 μm, optionally a Carr's Index less than about 25%, such as from about 18 to 24%, and optionally an MMAD from about 0.1 to 10 μm.

In one embodiment of the invention, the microparticles comprise an immunosuppressant as defined above and have a tap density from about 0.8 to 0.21 or 0.1 to 0.2 g/cm$^3$ and a median geometric diameter (preferably X50 or D50) of less than about 10 μm, such as from about 1 to 5 μm or from 1.5 to 4.5, or 1.75 to 4 μm or 2 to 3 or 3.5 μm, or 2 to 2.5 or 2.7 μm, optionally a Carr's Index less than about 30%, such as from about 20 to 28%, and optionally an MMAD from about 0.1 to 5 or 10 μm such as from 2 to 4 or 2 to 3 μm.

The microparticles may be suitable for *ex vivo* or *in vivo* administration to a mammal. The term mammal may include a human, as well as animals, such as cats, dogs and horses, preferably human. The administration may be by any route, including parenteral, such as by injection. It is preferred that the microparticles of the invention are suitable for oral or nasal inhalation.

In one embodiment, the microparticles are pharmaceutically acceptable. The microparticles may be sterile and optionally pyrogen-free. This may be required, for example, in injection.

In one embodiment of the invention, there is provided a pharmaceutical composition comprising the microparticles produced according to the invention. The composition may optionally comprise one or more pharmaceutically acceptable excipients or carriers, such as defined herein. The composition may, for example, be in the form of a solid, such as a powder, a liquid or a suspension of the microparticles in a non-solvent.

In the invention, the microparticles are obtainable by spray-drying in the presence of a blowing material as defined herein.

Procedures for preparing microparticles by spray-drying are fully described in, *inter alia,* WO 92/18164 and WO 96/15814. According to the present invention, these procedures are modified to produce the microparticles of the invention.

The microparticles are porous. The walls of the microparticles of the invention may comprise pores, such as, for example, gaps, voids, spaces, or fissures. In one embodiment of the invention, the pores may range in size from about 20 to about 1000 nm, such as from 400 to 800 nm.

In one embodiment, the microparticles comprise one or more walls. The wall or walls of the microparticles may be porous. For example the wall or walls of the microparticles may be porous as described in WO 98/17257. The microparticles according to the invention may have a wall thickness of no more than 500 nm, such as from about 10 to 250 nm, or from about 100 to 150 nm.

The microparticles of the invention, as described in any of the embodiments herein, are preferably in the form of a solid, such as a powder, preferably a spray-dried powder. The powder may be dry. By the term "dry" it is intended to mean that the liquid, such as water or moisture, content of the powder is less than about 15 wt.% based on the weight of the powder or less than about 10 wt.%, such as less than about 5 wt.%, for example, the powder may have a moisture content of from about 1 to 15 wt.%, or from 5 to 10 wt.%. For example, the powder may have a moisture content of from about 1 to 15 wt.%, from about 5 to 13 wt.%, from about 6 to 12 wt.% or from about 7, 8 or 9 to 10 wt.%.

In one embodiment, the moisture content of the powder may be less than about 5 wt.%, 1 wt.%, 0.5 wt.% or 0.1 wt.%. For example, the powder may have a moisture content of from about 0.01 to 5 wt.%, from about 0.1 to 4 wt.%, from about 0.5 to 3 wt.% or from about 0.75 to 1, 2 or 2.5 wt.%.

In one embodiment, the volatile content of the microparticles or powder (which can include, for example, solvents such as water and/or volatile solids) may be from about 0.01 to 10.0 wt.%, such as from about 0.1 to 8 wt.%, from about 0.5 to 7 wt.%, from about 1 to 6 wt.% or from about 3 or 4 to 5 wt.%. The volatile content may be measured using, for example, thermogravimetric analysis (TGA).

In one embodiment, the microparticles or powder, as described in any of the embodiments herein, provide a fine particle fraction (less than 6.5 $\mu$m or 5.8 $\mu$m) following aerosolization greater than about 25%, greater than about 35%, or greater than about 40% or 50% of the delivered dose. The Andersen Cascade Impactor or NGI may be used and the results analysed using Copley CITDAS software to determine the fine particle fraction and fine particle mass at different cut-off diameters e.g. <6.5 micron, <5.8 micron, <5 micron, <3.3 micron and <3 micron. The fine particle fraction is typically measured over a pressure drop of 4kPa.

In one embodiment of the invention, the fine particle fraction (less than 6.5 $\mu$m or 5.8 $\mu$m) is from about 20 to 90 %, from about 30 to 70 %, or from about 40 to 60%, such as from 70 or 80 to 90% of the delivered dose and/or the fine particle fraction (less than 5 $\mu$m) may be from about 30 to 60 %, or from about 40 to 50%, and/or the fine particle fraction (less than 3.3 or 3 $\mu$m) may be from about 10 to 90 %, from about 15 to 40 or 50 %, or from about 20 to 30% of the delivered dose.

In one embodiment of the invention, microparticles comprising an immunosuppressant, with an excipient, provide a mean fine particle fraction (less than 5.8 $\mu$m) of greater than 75%, 80% or 85%, such as from 75% to 90% or from 79 to 88% following aerosolisation from a dry powder inhaler, such as a monohaler.

In one embodiment of the invention, the microparticles are suitable for filling into a blister or other receptacle, or reservoir by machine or automated filling. The microparticles of the invention may be adapted for machine filling or automated filling. The microparticles may also have improved emptying performance from a blister, reservoir or other receptacle compared to microparticles not produced according to the invention.

The microparticles of the invention may be hollow i.e. comprise one or more voids, filled with gas or air, with a surrounding wall-forming material. The wall-forming material may comprise the saccharide as described herein. In a preferred embodiment, the hollow microcapsules are not honeycombs as in maltesers.

The term "microparticles" means, in one embodiment, hollow particles enclosing a space, which space is filled with a gas or vapour but not with any solid materials. Honeycombed particles resembling the confectionery sold in the UK as "Maltesers" (Regd TM) are not formed. It is not necessary for the space to be totally enclosed (although this is preferred) and it is not necessary for the microparticles to be precisely spherical, although they are generally spherical. If the microparticles are not spherical, then the diameters referred to above relate to the diameter of a corresponding spherical microparticle having the same mass and enclosing the same volume of hollow space as the non-spherical microparticle.

The microparticles of the invention are preferably hollow particles comprising at least one wall enclosing one or more spaces, more preferably one wall enclosing one space.

In one embodiment, the microparticles of the invention are water-soluble i.e., have a solubility of at least about 0.1 mg/cm$^3$ in water at a temperature of 20°C, at least about 0.5 mg/cm$^3$, or at least about 1.0 mg/cm$^3$.

The microparticles of the invention may be used in medicine. For example, the microparticles may be suitable for therapeutic or diagnostic use. The diagnostic use may include the use of the microparticles in ultrasonic imaging, for example as echogenic contrast agents.

In one aspect of the invention, the microparticles or powder may be used in the manufacture of a medicament for the treatment of a mammal in need thereof, such as a human. The term "mammal" also includes veterinary animals such as for example, horses, cows, sheep and pigs, as well as pets such as, for example, dogs, cats and hamsters. The condition to be treated may be one or more of asthma, chronic obstructive pulmonary disease (COPD), bronchitis, cystic fibrosis and other lung diseases, as well as acute and/or chronic lung transplant rejection and/or BO and/or BOS.

The microparticles of the invention comprising a saccharide such as heparin sodium, may be used in the treatment of one or more of the following conditions:

adult respiratory distress syndrome; allergic encephalomyelitis; allergic rhinitis; arthritis; asthma; cancer; delayed type hypersensivity reactions; inflammatory bowel disease; interstitial cystitis; respiratory disorder or disease which comprises increased levels and/or viscosity of mucus or other pulmonary secretions, such as CAL, pneumonia, sinusitis, sinus congestion, cystic fibrosis and asthma, where the subject to be treated may have a respiratory tract infection, such as a bacterial or viral infection, for example, influenza or a cold; chronic airflow limitation (CAL) with mucus hypersecretion; a disorder characterized by the presence of endogenous extracellular DNA, such as cystic fibrosis, CAL, pneumonia or systemic lupus erythematosus (SLE); and transplant rejection.

The microparticles can be used for facilitating the clearance of mucus from the central and peripheral airways of a human subject with chronic airflow limitation (CAL) who has mucus hypersecretion.

The microparticles may also be used for the treatment of a pulmonary disease, such as, for example, a pulmonary disease involving hypersecretion of mucus or abnormal viscoelasticity of mucus.

In one embodiment, the pulmonary disease is selected from one or more of chronic bronchitis, acute asthma, cystic fibrosis (CF), chronic obstructive pulmonary disease (COPD) or bronchiectasis.

The microparticles may be used for the treatment of the following conditions: as an anticoagulant, preventing the formation of clots and extension of existing clots within the blood; for anticoagulation for the following conditions: acute coronary syndrome, e.g., NSTEMI atrial fibrillation, deep-vein thrombosis and pulmonary embolism, cardiopulmonary bypass for heart surgery.

The microparticles comprising heparin and its derivatives (enoxaparin, dalteparin, and so forth) may be effective at preventing deep-vein thromboses and pulmonary emboli in patients at risk.

There is also disclosed a powder comprising microparticles according to any of the embodiments set out above. The powder is preferably free of excipients and additives as described above, or lubricants, such as surfactants, and/or preferably dry, as defined above.

In one embodiment, the powder is free-flowing. By "free-flowing", it is preferably intended to mean that the difference between the tap density and the bulk particle density of the microparticles is less than about 0.07 g/cm$^3$, less than about 0.05 g/cm$^3$, such as less than about 0.03 g/cm$^3$, for example from about 0 to 0.05 g/cm$^3$ or from about 0.01 to 0.03 g/cm$^3$ or that the powder has a Carr's Compressibility Index (CCI) of less than 30%, such as less than 25% or 20% or a Carr's Index of from about 5% to 30%, from about 10% to 25%, or from about 15 or 19 to 23% or from 20 or 21 to 29%, 22 or 23 to 27 or 24 or 25 to 26%.

In one embodiment, a container is provided comprising the powder or microparticles according to the invention. The container may be a capsule, blister, reservoir or other receptacle for housing the powder. The powder or microparticles are preferably in the form of a plug in the container. The plug is typically friable and may have a CCI as defined above or produce a fine particle fraction as defined above.

The evacuation of the powder according to the invention from a container, such as a blister, comprising the powder can be greater than about 70 wt.% based on the fill weight, such as greater than about 80 wt.% or greater than about 95 wt.%, for example from about 80 to 99 wt.%, from about 85 to 98 wt.%, or from about 90 to 95 wt.%.

The low density of the microparticles can provide less weight for the same volume and therefore allow a low dose to be provided in a container without a carrier.

Microparticles may be suitable for formulation in an inhaler. If they comprise a therapeutic agent, they provide rapid release and subsequent uptake of drug in the lung. Further, the microparticles and powders may not require a carrier for effective administration to the lung. An inhaler including microparticles may therefore contain the microparticles as the sole or predominant component of the inhalable formulation, for example, greater than about 80, 90 wt.% or 95 wt.%, such as from about 80 to 100 wt.%, or from about 85 to 95 wt.%.

For administration by inhalation, the water-soluble microparticles obtained by spray-drying are preferably used. Stabilisation may be used, if another route of administration is required and/or for diagnostic purposes. The amount of microparticles to be administered can readily be determined by the skilled man.

The powders may be suitable for systemic or topical delivery via pulmonary or nasal routes. The microparticles may be used in conjunction with an inhalation device such as a metered dose inhaler, a dry powder inhaler or a nebulizer. For example, an inhaler may comprise microparticles or a powder according to the invention. The inhaler may be a metered dose inhaler, a dry powder inhaler or a nebulizer. In one embodiment, the inhaler is a dry powder inhaler, such as defined herein.

The microparticles which include a medicament, for example one or more of the agents described above, can be administered to the respiratory tract of a mammal in need of treatment, prophylaxis or diagnosis. Administration of particles to the respiratory system can be by means known in the art. For example, particles can be delivered from an inhalation device. In a preferred embodiment, particles are administered via a dry powder inhaler (DPI). Metered-dose inhalers (MDI), or instillation techniques, also can be employed.

Various suitable devices and methods of inhalation which can be used to administer particles to a mammal's respiratory tract are known in the art. Suitable inhalers include the Monohaler and Dinkihaler.

One example of an inhalation device, or inhaler, is a pressurized metered dose inhaler, a device which produces the aerosol clouds for inhalation from solutions and/or suspensions of respiratory drugs in chlorofluorocarbon (CFC) and/or hydrofluroalkane (HFA) solutions. The metered dose inhaler can be a soft mist inhaler (SMI), in which the aerosol cloud containing a respiratory drug can be generated by passing a solution containing the respiratory drug through a nozzle or series of nozzles. The aerosol generation can be achieved in SMI, for example, by mechanical, electromechanical or thermomechanical process. Examples of suitable soft mist inhalers include the Respimat® Inhaler (Boeringer Ingelheim GmbH), the AERx® Inhaler (Aradigm Corp.), the Mystic™ Inhaler (Ventaira Pharmaceuticals, Inc) and the Aira™ Inhaler (Chrysalis Technologies Incorporated).

Other passive or active dry powder inhalers may alternatively be employed. In one version, a passive dry powder inhaler is preferred because of its ease of use and reproducible aerosolization. Suitable passive dry powder inhalers include both capsule-based inhalers and blister-based inhalers. Capsule-based passive inhalers are particularly preferred due to their larger unit dose volume (compared to current blister devices), which facilitates higher lung doses per puff.

Devices sold or marketed under the following tradenames and/or trademarks may also be suitable: Handihaler (Boehringer Ingelheim), Eclipse (Aventis), AIR inhaler (Alkermes), Cyclohaler (Plastiape), Concept 1 (Novartis), Flowcaps (Hovione), Turbospin (PH&T), Monohaler (Pfizer), Spinhaler (Aventis), Rotahaler (GSK). Suitable blister-based inhalers include: the Diskus and Gemini (GSK), the device of Nektar Therapeutics disclosed in PCT Application No. US2007/022830, Gyrohaler (Vectura), E-Flex, Microdrug, Diskhaler (GSK). Also within the scope of the present invention are active dry powder inhalers including: the Exubera® inhalation device, which is described in U.S. Patent No. 6,257,233, Aspirair (Vectura), and Microdose inhaler (Microdose).

With regard to doses for multi dose dry powder inhalers, the inhalers can be configured to provide any suitable number of doses, typically between about 30 - 120 doses and more typically between about 30-60 doses. The inhalers can deliver one drug or a combination of drugs. In some embodiments the inhalers can provide between about 30-60 doses of two different drugs (in the same or different unit amounts), for a total of between about 60-120 individual unit doses, respectively. The inhaler can provide between a 30 day to a 60 day (or even greater) supply of medicine. In some embodiments the inhalers can be configured to hold about 60 doses of the same drug or drug combination, in the same or different unit amounts, which can be a 30 day supply (for a twice per day dosing) or a 60 day supply for single daily treatments.

Other suitable inhalers include, for example, unit-dose preloaded/reloadable and multidose dry powder inhalers.

The inhaler may comprise the device described in PCT/EP2010/050790.

The microparticles and/or powders including those comprising a saccharide and an immunosuppressant, may further comprise and/or be coprocessed with a force control agent (FCA), which include an amino acid such as, for example, leucine, or a surface active material such as, for example, lecithin and magnesium stearate. The force control agents preferably exhibit anti-adherent and/or anti-friction properties, such as to reduce the attractive force between saccharide or immunosuppressant particles and excipient particles. One or more force control agents may be used.

The force control agent is preferably in the form of particles. Advantageously, at least 95 wt. % of the particles have a mass median aerodynamic diameter less than 150$\mu$m, more advantageously less than 100$\mu$m, preferably less than 50$\mu$m. In one embodiment, the mass median aerodynamic diameter of the additive particles is not more than about 10 $\mu$m.

In one embodiment, the force control agent is selected from leucine, lecithin and magnesium stearate in the form of particles.

The force control agent may be present in an amount of less than 10 wt.% based on the weight of the microparticles or powder, more advantageously not more than 5 wt.%, such as not more than 4 wt.% or not more than 2 wt.%

or less than 1.5 wt.% or less than 1.4 wt.% such as less than 1 wt.% or from about 0.1 to 1.3 wt.%.

The force control agent may include one or more compounds selected from amino acids and derivatives thereof, and peptides and polypeptides having molecular weight from 0.25 to 1000 KDa, and derivatives thereof.

In one embodiment, the force control agent comprises an amino acid. The agent may comprise, for example, one or more of any of the following amino acids: leucine, isoleucine, lysine, valine, methionine, phenylalanine. The agent may be a salt or a derivative of an amino acid, for example aspartame or acesulfame K. Preferably the agent is selected from particles which consist substantially of leucine, advantageously L-leucine. The L- forms and the D- and DL-forms may be used.

Alternatively, the force control agent may comprise particles of a phospholipid or a derivative thereof. Lecithin has been found to be a good material for the agent.

The force control agent may include or consist of one or more surface active materials, in particular materials that are surface active in the solid state, which may be water soluble, for example lecithin, in particular soya lecithin, or substantially water insoluble, for example solid state fatty acids such as lauric acid, palmitic acid, stearic acid, erucic acid, behenic acid, or derivatives (such as esters and salts) thereof. Specific examples of such materials are: magnesium stearate; sodium stearyl fumarate; sodium stearyl lactylate; phospatidylcholines, phosphatidylglycerols and other examples of natural and synthetic lung surfactants; Liposomal formulations; lauric acid and its salts, for example, sodium lauryl sulphate, magnesium lauryl sulphate; triglycerides such as Dynsan 118 and Cutina HR; and sugar esters in general.

Coprocessing of the microparticles or powder of the invention with one or more agents may be conducted using mechanofusion-a dry mechanical fusion process such as described in the Journal of Pharmaceutical Sciences 2009, vol. 98, n°8, pp. 2770-2783 .

Mechanofusion is a dry process designed to mechanically fuse a first material onto a second material. It should be noted that the use of the terms "machanofusion" and "mechanofused" are supposed to be interpreted as a reference to a particular type of milling process, but not a milling process performed in a particular apparatus. The compressive milling processes work according to a different principle to other milling techniques (Comminution techniques), relying on a particular interaction between an inner element and a vessel wall, and they are based on providing energy by a controlled and substantial compressive force.

In the process of the invention, the agent, saccharide or immunosuppressant may be as defined in any of the above embodiments. The microparticles produced may also be as defined in any of the above embodiments.

The term "blowing material" as used herein refers to ammonium carbonate or ammonium bicarbonate.

By way of example, the blowing material used in the production of heparin microparticles is ammonium carbonate or ammonium bicarbonate which releases ammonia, carbon dioxide and water vapour. During spray-drying, these three gases expand in the atomised droplets, causing the droplet to increase in size, to produce larger microparticles.

In one embodiment of the invention, the blowing material is not retained in the microparticles. For example, the blowing material or any residue thereof is preferably present in the microparticles an amount of less than about 2 wt.%, such as less than about 1 wt.%, for example less than about 0.1 wt.%, 0.01 wt.%, or 0.001 wt.% or about 0 wt.%.

The blowing material is selected from ammonium carbonate and ammonium bicarbonate or mixtures thereof.

The solution or dispersion used in the process is preferably substantially free of excipients or additives. However, excipients or carriers to be included in the microparticles (i.e. not the carrier for the solution or dispersion) may be present, for example where an immunosuppressant is used in the feedstock. In this case, the excipients and carriers may be as defined herein. Additives and excipients preferably include any additional material, other than saccharide as defined herein and carrier, and include, for example, amino acids such as leucine, buffers or salts, for example, phosphate or citrate, surfactants, such as non-ionic surfactants, polymers and/or phospholipids such as DPPC. By the term "substantially free", it is intended to mean that the excipient or additive is present in an amount of less than about 10% w/w based on the weight of the carrier, such as less than about 5% w/w, less than about 2% w/w, or less than about 1% w/w, such as less than about 0.1% w/w or 0.01% w/w or about 0% w/w.

In one embodiment of the invention, where an immunosuppressant is present, then the solution or dispersion does not comprise a wetting agent, such as for example polyethylene glycols (PEGs), and/or a surfactant, such as TWEEN, and/or a chlorinated solvent, such as methylene chloride.

The pH of the solution or dispersion may be from about 1 to 12, such as from about 2 to 10, for example from about 3 to 9 or about 4 to 8. In one embodiment, the pH of the solution or dispersion is less than about 7, for example from about 2 to 6. In another embodiment of the invention, the pH of the solution or dispersion is greater than about 7, for example from about 9 to 12.

In an embodiment of the process, the carrier for the solution or dispersion is an aqueous carrier or an aqueous-organic carrier i.e., comprising an organic solvent, such as an alcohol or other carbon-containing solvent, which can include chlorinated organic solvents. The aqueous carrier or aqueous-organic carrier preferably comprises water in an amount of at least about 50% v/v based on the total volume of carrier, such as greater than about 60% v/v, 70% v/v, 80% v/v or 90% v/v, for example, from about 50 to 99% v/v, from about 60 to 95% v/v, or from 70 to 90% v/v or from about 80 to 90% v/v.

In one embodiment of the process, where an immunosuppressant is used, the carrier for the solution or dispersion is non-chlorinated or does not comprise a chlorinated solvent such as methylene chloride.

The carrier, such as an aqueous carrier, does not comprise a liquid of greater volatility than water, for example a volatile liquid having a boiling point lying between 20°C and 100°C, such as an alcohol, for example ethanol or methanol or a ketone such as acetone, or comprises the liquid, such as an alcohol, in an amount of less than about 20% v/v, such as less than about 10% v/v.

The solution or dispersion preferably comprises the saccharide as defined herein, in a concentration of less than or about 40%, 30% or 20% w/v or less than or about 10% w/v based on the volume of the carrier, such as from about 1 to 15% w/v, from about 2 to 10% w/v, or from about 3 to 8% w/v.

The solution or dispersion preferably comprises the blowing material, as defined herein, in a concentration of less than or about 40%, 30 or 20% w/v or less than or about 10% w/v based on the weight of the carrier, such as from about 1 to 15% w/v, from about 2 to 10% w/v, or from about 3 to 8% w/v.

In one embodiment, the total amount of the feedstock for the carrier for the solution or dispersion (comprising the saccharide and the blowing material, together with any optional components such as excipients) is from about 1 to 30 % w/v based on the volume of the carrier, such as from 2 to 20 or 3 to 10% w/v.

The weight ratio of saccharide to blowing material is preferably from about 5:1 to about 1:5, 4:1 to about 1:4 or 3:1 to about 1:3, more preferably from about 2:1 to about 1:2, such as from about 2:1 to about 1:1.5 or about 1:1.

In one embodiment, the solution or dispersion comprises the saccharide and blowing material each in a concentration of from about 1 to about less than 10 % w/w and with a weight ratio of saccharide to blowing material of from about 3:1 to about 1:1.

In one embodiment, the solution or dispersion comprises the saccharide and blowing material each in a concentration of less than about 10 % w/w and with a weight ratio of saccharide to blowing material of from about 3:1 to about 1.1:1.

The process of the invention may be carried out on any suitable spray drying apparatus. A suitable apparatus is, for example, the Standard Niro Mobile Minor Spray Dryer.

The atomisation may be carried out using a suitable nozzle, such as a two-fluid nozzle. The atomization pressure of the apparatus is preferably greater than 2 bar g, such as from about 2 to 10 bar g, from about 3 to 6, 7 or 8 bar g, such as from about 4, 5 or 6 to 7 bar g.

The inlet temperature of the apparatus may be from about 100 to 250°C, or from about 140 to 220°C, such as from about 150 to 210°C. The outlet temperature of the apparatus is generally lower than the inlet temperature. Preferably, the outlet temperature is from about 30 to 110°C, such as from about 50 to 100°C, for example from about 70 to 90°C.

In one embodiment of the invention, where an immunosuppressant is present in the solution or dispersion, such as tacrolimus, with an excipient, such as trehalose, the atomisation pressure is from about 3 to 6 bar and/or the inlet temperature is from about 100 to 150°C and/or the outlet temperature is greater than about 75°C.

In the process of the invention, the above parameters may be used in any combination.

In one embodiment of the invention, the blowing material may be removed, for example from the atomised solution or dispersion, by evaporation or vaporization. Alternatively, the blowing material may decompose during or after atomisation and the release of a gas or gases lead to the formation of the microparticles. The removal and/or decomposition of the blowing material may or may not be substantially simultaneous with evaporation of the carrier.

In one aspect, the invention provides the use of a blowing material, which is ammonium carbonate or ammonium bicarbonate, in the formulation of microparticles by spray drying to aid the automated and/or machine filling of therapeutic microparticles or powders such as according to the invention into a receptacle and/or the emptying of therapeutic microparticles or powders such as according to the invention from a receptacle.

The therapeutic microparticles or powders may comprise one or more saccharide as defined herein. For example, the therapeutic microparticles or powder may comprise a glycosaminoglycan, preferably unfractionated, selected from one or more of heparin, heparin sulphate, heparitin sulphates, such as heparan sulphate proteoglycan, heparinoids, dermatan, dermatan sulphate, keratin, chondroitin, chondroitin sulphates A, B, C, D and E, heparan, heparan sulphate, keratan sulphate, hyaluronic acid, physiologically acceptable salts thereof, derivatives or fragments thereof, or mixtures of any thereof, preferably the sodium salt of heparin (heparin sodium) or heparin sulfate.

For example, a blowing material may be used to increase the delivered dose for a powder, preferably compared to the same powder which has not been prepared using the blowing material.

The microparticles or powder preferably comprise a polysaccharide, such as, for example, heparin sodium or heparin sulphate or an immunosuppressant, such as tacrolimus, sirolimus, pimecrolimus or a derivative thereof, and optionally a pharmaceutically acceptable excipient or carrier, such as a saccharide, amino acid, a sugar alcohol or a mixture thereof. The microparticles or powder are "blown" i.e. prepared using a blowing material as defined herein.

In one embodiment, the use of a blowing material, in a solution or dispersion to be atomised, produces microparticles as defined herein, preferably in the form of a powder, for which filling into and/or emptying from a receptacle, such as a blister or capsule, is enhanced compared to microparticles or powders which have not been prepared using the blowing material in the solution or dispersion to be atomised. This can overcome problems seen when trying to fill and aerosolize otherwise cohesive powders.

All combinations of the parameters set forth above, including median geometric diameter, mass median aerodynamic diameter, tap density, bulk density and Carr's Index, as well as the agents, immunosuppressants and saccharides mentioned, are contemplated and encompassed by the present invention.

The listing or discussion of an apparently prior-published document in this specification should not necessarily be taken as an acknowledgement that the document is part of the state of the art or is common general knowledge.

The following non-limiting examples illustrate the invention and do not limit its scope in any way. In the examples and throughout this specification, all percentages, parts and ratios are by weight unless indicated otherwise. Average molecular weights are based on weight unless otherwise specified. It will be appreciated that the various percentage amounts of the different components that are present in the products of the invention, including any optional components, will add up to 100%.

EXAMPLES:

Ammonium carbonate was added to solutions of heparin in water in ratio's of 1: 0.75, 1:1 and 1:2 w/w (heparin: ammonium carbonate). Each batch was spray dried using the Niro Mobile Minor spray dryer. The particle size, Carr's Index and aerosol performance from the Monohaler inhaler using Andersen Cascade Impactor analysis was determined.

Example 1:

A 1:1 solution of 5% heparin: 5% ammonium carbonate (w/w) was spray dried on the Niro Mobile Minor spray dryer. An atomization pressure of 6 bar and an atomization flow rate of 10 L/sec were used.

Equipment/Reagents:

- hyclone WFI Water
- Ammonium carbonate
- Heparin Sodium

Method:

Approximately 5g of heparin was weighed and transferred into a 500ml glass vessel. 100mL water was added and the solution mixed until complete dissolution occurred. To this solution, 5g of ammonium carbonate was added. This solution was then spray dried as described below.

Spray Drying Conditions:

**Feed Material**

| Material / Type | Concentration (% w/w) | Volume (ml) | Mass Spray Dried (g) | Additions | Feed Rate (g/min) |
|---|---|---|---|---|---|
| Heparin | 5 | 100 | 5 | 5% ammonium carbonate (5g) | 20 |

**Drying Conditions**

| Inlet Temperature (°C) | Outlet Temperature (°C) | | Atomisation Type | Atomisation Pressure (bar g) | Atomisation Airflow (l/s) |
|---|---|---|---|---|---|
| | START | FINISH | | | |
| 200 | 78 | 78 | 2FN | 6 | 10 |

Particle Size Analysis

The particle size distribution of spray dried heparin with ammonium carbonate (1:1) 5% - (RHP081201AKSA) was analysed using a Laser Diffraction Particle Size Analyser (Sympatec, Clausthal-Zellerfeld, Germany). A R2 lens was used with a 1.0 bar dispersion pressure.

Results:

Table 1

| | $X_{10}$ (μm) | $X_{50}$ (μm) | $X_{90}$ (μm) | $X_{99}$ (μm) |
|---|---|---|---|---|
| Mean | 1.29 | 3.59 | 7.80 | 17.11 |
| SD | 0.04 | 0.04 | 0.08 | 0.23 |
| % RSD | 3.14 | 1.16 | 1.03 | 1.36 |

**Example 2:**

A comparison of data generated when heparin loading and spray drying conditions are kept constant but the ammonium carbonate loading is increased is shown in Table 2.

EP 2 429 497 B1

Table 2

**Characterisation of Heparin Batches**

| Batch Number | Heparin loading (%) | Ammonium carbonate loading (%) | Aerosol performance (% Fine Particle Fraction) | | Bulk Density g/cm3 | Tapped Density (g/cm3) | Carrs Compressibility Index (%) | Particle size (X50, µm) |
|---|---|---|---|---|---|---|---|---|
| | | | <6.5µm | <3µm | | | | |
| RHP081021AKSA | 10 | 7.5 | 38.2 | 18.5 | 0.11 | 0.14 | 21 | 4.8 |
| RHP081020AKSB | 10 | 10 | 29.7 | 12.3 | 0.05 | 0.05 | 18 | 9.0 |
| RHP081110ANHB | 5 | 3.75 | 44.2 | 23.1 | 0.12 | 0.19 | 35 | 2.6 |
| RHP081112ANHA | 5 | 5 | 44.8 | 20.3 | 0.08 | 0.11 | 29 | 4.2 |

The data in Table 2 surprisingly show that when the reaction conditions are kept constant, in particular, when the amount of agent, such as heparin, is less than 10 % w/w, then the amount of blowing material, such as ammonium carbonate, can actually be reduced or increased (so that the weight ratio of agent to blowing material increases from 1:1 to 2:1 or decreases from 2:1 to 1:1) without substantially affecting the fine particle fraction and despite the change in particle size. Thus, it has been found possible to make larger particles (4.2$\mu$m) behave as though they were much smaller in terms of their aerosolization behaviour, and so produce a high fine particle fraction, even though the size of the particles increases.

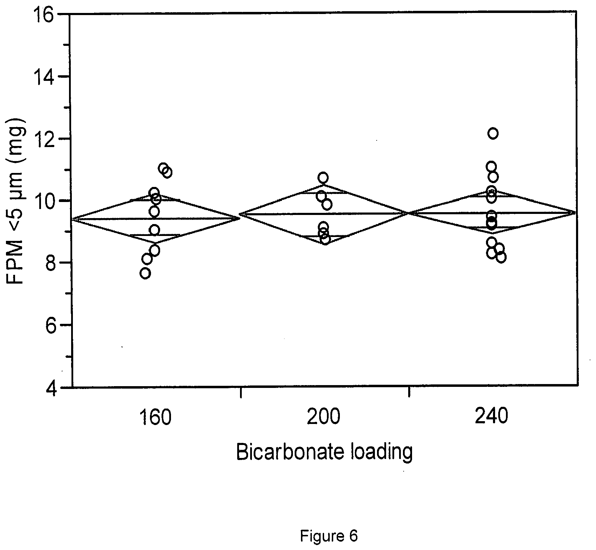

Figure 6 shows that increasing or decreasing the bicarbonate level does not significantly affect the FPM (<5$\mu$m).

## Example 3:

### Filling of low density formulation and standard heparin: leucine formulation

Automated drum (Omnidose) filling of spray dried powders can be difficult due to the level of powder compaction required to achieve a consistent fill weight. As a result, poor aerosol perfomance is often seen when the compacted plugs are aerosolised in an inhaler.

Studies have been carried out to evaluate if the low density (fluffy) formulation according to the invention (ammonium carbonate, 1:1 ratio) remains more friable following drum filling compared to the standard heparin/leucine formulation.

It can be seen that the plugs formed following filling of the low density powder according to the invention gives rise to plugs with far greater friability than those obtained with the standard formulation friable plugs. This is confirmed by the better blister evacuation seen with the low density formulations in Table 3.

Table 3

| Filling Process / Blend | Drum Size (mm$^3$) | Mean Fill Weight (mg) $\pm$ stdev | Mean Shot Weight (mg) $\pm$ stdev | **Blister Evacuation % $\pm$ stdev** |
|---|---|---|---|---|
| Drum filling of formulation without blowing agent | 16.2 | 14.0 $\pm$ 1.2 | 7.8 $\pm$ 4.2 | **56 $\pm$ 29.6** |
| Drum filling of heparin with blowing agent | 65.2 | 4.4 $\pm$ 0.9 | 3.4 $\pm$ 0.3 | **76 $\pm$ 7.1** |

## Example 4:

Ammonium Bicarbonate Evaluation (33 mg fill weight).

Bicarbonate was used in place of carbonate in the process described in Example 1.

It has been unexpectedly found in the present invention that the use of ammonium bicarbonate, under the same spray-drying conditions, can result in an improvement is delivered dose and fine particle fraction over ammonium carbonate (see Table 4 below).

Table 4

| Parameter | Heparin without Blowing agent | Carbonate Blowing Agent | Bicarbonate Blowing Agent |
|---|---|---|---|
| Particle size (X50, $\mu$m) | 2.1 | 3.9 | 4.2 |
| Moisture content (%) | ~9 | 9.2 | 8.9 |
| Metered dose | 25.7 | 24.7 | 27 mg |
| Delivered dose (mg) | 22.8 | 20.7 | 25 mg |
| FPM* < 6.5 (mg) | 10.9 mg (48) | 11.1 mg (54) | 14.0 mg (58) |
| FPM* <5 (mg) | 9.3 mg (41) | 8.8 mg (43) | 11.3 mg (46) |
| FPM* < 3 (mg) | 5.6 mg (25) | 5.1 mg (25) | 6.5 mg (26) |

(continued)

| Parameter | Heparin without Blowing agent | Carbonate Blowing Agent | Bicarbonate Blowing Agent |
|---|---|---|---|
| MMAD ($\mu$m) | 3.7 | 4.8 | 4.7 |
| Bulk density (g/cm3) | 0.6 | 0.11 | 0.08 |
| Tapped density (g/cm3) | 0.9 | 0.14 | 0.10 |
| CCI (%) | 32 | 25 | 21 |
| *Fine particle fraction (%) values shown in parentheses | | | |

### Example 5:

A summary comparing a spray dried heparin batch manufactured using the same process parameters as those used for microparticles according to Example 1 is provided in Table 5.

For the spray dried heparin batch (which has been prepared without the use of a blowing material i.e. it is non-engineered), the aerosol performance is very poor with significantly lower fine particle masses (FPM). This is due to the fact that most of the powder is retained in the device - this is supported by the fact that the delivered dose is much lower than the metered dose.

The particles according to the invention are lighter and larger than the comparative heparin/leucine particles but still produce high fine particle fractions.

### Comparative Example 6:

Example 1 in WO 99/32083 was repeated using the same amount of heparin sodium in the place of HSA. The results of the experiment are provided in Table 6. As shown in Table 6, a large geometric diameter is found (15$\mu$m) and a low bulk density (0.01 g/cm$^3$).

The aerosol performance of this comparative formulation is less than that of the formulation according to the present invention. Thus, the fine particle fractions for the comparative formulation are almost half, for <6.5$\mu$m and <5$\mu$m, as shown in Table 6, compared to the microparticles according to the invention.

Table 5

| Batch No. | ACI data (mg) | | | | | | | | | | Particle Size Data | | | Density | | Carr's Index (%) |
| | FPM ≤ 6.5 μm | FPF (%) | FPM ≤ 5 μm | FPF (%) | FPM ≤ 3 μm | FPF (%) | Metered dose | Delivered dose | MMAD μm | Mass balance (%) | D10 | D50 | D90 | Bulk (g/ml) | Tapped (g/ml) | |
|---|---|---|---|---|---|---|---|---|---|---|---|---|---|---|---|---|
| Heparin only RHP090203ANHA | 5.16 | 72.1 | 4.87 | 68.2 | 4.07 | 56.9 | 27.62 | 7.15 | 2.3 | 94.6 | 0.67 | 1.35 | 2.49 | 0.4197 | 0.5644 | 25.6 |
| | 7.15 | 68.3 | 6.94 | 66.2 | 5.48 | 52.4 | 25.58 | 10.47 | 2.4 | 87.4 | 0.70 | 1.38 | 2.53 | | | |
| | 7.32 | 71.9 | 7.02 | 69.0 | 5.67 | 55.7 | 27.09 | 10.18 | 2.3 | 91.6 | | | | | | |
| | **6.54** | **70.8** | **6.28** | **67.8** | **5.07** | **55.0** | **26.76** | **9.27** | **2.3** | **91.2** | **0.69** | **1.37** | **2.51** | | | |
| Heparin Leucine | 10.29 | 45.5 | 86.57 | 38.3 | 5.16 | 22.8 | 25.76 | 22.61 | 3.8 | 93.0 | 0.70 | 2.17 | 4.99 | 0.6169 | 0.9130 | 32.4 |
| | 11.15 | 49.2 | 94.60 | 41.7 | 5.80 | 25.6 | 26.98 | 22.68 | 3.6 | 97.4 | 0.69 | 2.29 | 5.06 | | | |
| | 11.28 | 48.8 | 95.66 | 41.4 | 5.90 | 25.5 | 24.47 | 23.13 | 3.7 | 88.4 | 0.70 | 2.33 | 5.11 | | | |
| | **10.91** | **47.8** | **92.28** | **40.5** | **5.62** | **24.6** | **25.74** | **22.81** | **3.7** | **92.9** | **0.70** | **2.26** | **5.05** | | | |
| Heparin Bicarbonate RHP0901160MB | 14.34 | 57.0 | 11.49 | 45.6 | 6.32 | 25.1 | 26.78 | 25.18 | 4.6 | 99.1 | 1.53 | 4.09 | 10.02 | 0.0866 | 0.1082 | 20.0 |
| | 13.63 | 53.9 | 11.14 | 44.0 | 6.65 | 26.3 | 27.55 | 25.31 | 4.8 | 101.8 | 1.65 | 4.25 | 10.37 | 0.0765 | 0.0994 | 23.0 |
| | | | | | | | | | | | 1.66 | 4.27 | 10.35 | 0.0850 | 0.1063 | 20.0 |
| | **13.99** | **55.4** | **11.32** | **44.8** | **6.49** | **25.7** | **27.17** | **25.25** | **4.7** | **100.5** | **1.61** | **4.20** | **10.25** | **0.0827** | **0.1046** | **21.0** |

Table 6

| Batch No. | ACI data (mg) | | | | | | | | | | Particle Size Data | | | Bulk (g/ml) | Tapped (g/ml) | Carr's Index (%) |
|---|---|---|---|---|---|---|---|---|---|---|---|---|---|---|---|---|
| | FPM≤ 6.5 μm | FPF (%) | FPM≤ 5 μm | FPF (%) | FPM≤ 3 μm | FPF (%) | Metered dose | Delivered dose | MMAD μm | Mass balance (%) | D10 | D50 | D90 | | | |
| Heparin:ammonium carbonate | 6.17 | 27.47 | 4.03 | 17.94 | 2.06 | 9.17 | 23.24 | 22.47 | 7.9 | 84.8 | 4.70 | 16.28 | 43.30 | 0.0114 | 0.0158 | 27.8 |
| | 7.51 | 28.67 | 4.85 | 18.51 | 2.41 | 9.20 | 27.04 | 26.19 | 7.9 | 101.2 | 4.41 | 15.58 | 41.21 | 0.0126 | 0.0166 | 24.1 |
| | 7.22 | 29.54 | 4.39 | 17.96 | 1.88 | 7.71 | 25.43 | 24.44 | 7.9 | 94.9 | 4.32 | 15.63 | 41.29 | 0.0120 | 0.0162 | 25.9 |
| | **6.97** | **28.56** | **4.42** | **18.14** | **2.12** | **8.69** | **25.24** | **24.37** | **7.9** | **93.6** | **4.48** | **15.83** | **41.93** | **0.0120** | **0.0162** | **25.9** |

## Example 7:

Tacrolimus formulations have been manufactured using different levels of trehalose (0, 50 & 80%), as an excipient, and ammonium bicarbonate (0 & 20%). The percentage of ammonium bicarbonate added is based on the mass of tacrolimus and trehalose in the feedstock solution.

Tacrolimus was dissolved in ethanol. Trehalose and ammonium bicarbonate were dissolved in water and the resulting solutions mixed in a 1:1 ratio. Each batch was spray dried using a bespoke mini spray dryer.

| Feedstock | |
|---|---|
| Feedstock Concentration | 2.5 %w/v |
| Feed Rate | 5 g/min |
| **Drying Conditions** | |
| Atomisation Pressure | 5 bar |
| Atomisation Flow | 25 l/min |
| Drying Pressure | 1 bar |
| Drying Flow | 5 l/s |
| Inlet Temperature | 120 °C |
| Outlet Temperature | >75 °C |

Device testing and aerodynamic particle size analysis

The formulations were tested using a unit dose dry powder inhaler (DPI) device and were filled with formulation to deliver ∼1mg dose.

The device was tested at flow rate Q (∼60 l/min)

- Re-usable single unit dose device
- Dose contained in a replaceable Al blister The unit dose blisters sealed using a manually operated sealing machine
- Conditions: 160 °C / < 0.5 sec Particle size determined by Next Generation Impactor (NGI)
- Collection cups coated with Brij 30 (5% in Hexane)
- Recovery in 85% methanol / 15% water

Particle Size Analysis

The particle size distribution of the formulations was analysed using a Morphologi G3 Particle Image Analyser.

Table 7

| | 100% Tacrolimus 0% Trehalose Ammonium Bicarbonate (20%) | | 50% Tacrolimus 50% Trehalose | 50% Tacrolimus 50% Trehalose Ammonium Bicarbonate (20%) | | 20% Tacrolimus 80% Trehalose | |
|---|---|---|---|---|---|---|---|
| Batch Reference | RTA090 224PWA | RTA090 414YLA | RTA090 416YLA | RTA090 224YLA | RTA090 415YLA | RTA090 212PWA | RTA090 420YLA |
| Mean Tacrolimus delivered dose (μg)[1] | 833 | 769 | 790 | 956 | 790 | 888 | 864 |
| Mean FPM <5.8μm (μg) | 706 | 675 | 624 | 692 | 673 | 507 | 626 |

(continued)

| | 100% Tacrolimus 0% Trehalose Ammonium Bicarbonate (20%) | | 50% Tacrolimus 50% Trehalose | 50% Tacrolimus 50% Trehalose Ammonium Bicarbonate (20%) | | 20% Tacrolimus 80% Trehalose | |
|---|---|---|---|---|---|---|---|
| Batch Reference | RTA090 224PWA | RTA090 414YLA | RTA090 416YLA | RTA090 224YLA | RTA090 415YLA | RTA090 212PWA | RTA090 420YLA |
| Mean FPF <5.8μm (%) | 85 | 88 | 79 | 72 | 85 | 57 | 73 |
| MMAD (μm) | 2.3 | 2.1 | 2.4 | 2.8 | 2.1 | 3.9 | 2.8 |
| Particle size: | | | | | | | |
| D10 (μm) | 1.23 1.20[2] | 1.43 | 1.59 | 0.76 | 1.40 | 1.14 | 1.28 |
| D50 (μm) | 3.17 3.01[2] | 2.22 | 2.63 | 2.04 | 2.38 | 3.20 | 2.18 |
| D90 (μm) | 7.66 7.48[2] | 3.51 | 4.26 | 5.89 | 3.90 | 6.59 | 3.77 |
| Untapped density (g/cm$^3$) | - | 0.13 | 0.16 | - | 0.10 | | 0.17 |
| Tapped density (g/cm$^3$) | - | 0.17 | 0.23 | - | 0.13 | - | 0.25 |
| Carr's Index (%) | - | 25 | 33 | - | 21 | - | 32 |
| [1] ex-device calculated from NGI data [2] Measurements taken in duplicate | | | | | | | |

**Example 8:**

Summary of Physical Characterisation for Tacrolimus Formulations

TGA Analysis

Samples were held at 25°C for 1 min then heated from: 25-135°C at 50°C min[-1] for 100% tacrolimus and blown tacrolimus formulations; 25-150°C at 50°C min[-1] for formulations containing trehalose.

No mass loss effects were observed as a result of residual ammonium bicarbonate; both formulations manufactured with and without ammonium bicarbonate demonstrated a similar total mass loss to the 100% spray dried formulation (~0.6%).

An increased mass loss was observed in the formulation containing 50% trehalose (~2.8%), with a further increase in the formulation containing 80% trehalose (~6.2%).

DSC Analysis

Samples were cooled to 0°C before being heated to 140°C at a rate of 50°C min[-1].

Two observations of interest were made during analysis. The first was the presence of an endotherm within the spray dried tacrolimus material (~85°C). This was unexpected in an amorphous material. This endotherm was present in all formulations.

The second observation was that of a glass transition associated with trehalose at ~50°C. This appeared at a lower temperature than would be expected of amorphous trehalose (~115°C). The formulations containing the trehalose also demonstrated the endotherm observed for tacrolimus, originally with an onset temperature of ~85°C, but at a lower onset temperature of ~70°C.

## Example 9

Product Stability Data

The following spray dried formulations were examined:

(1) 100% Tacrolimus (prepared with 20% ammonium bicarbonate)
(2) 50% Tacrolimus / 50% Trehalose (prepared with 20% ammonium bicarbonate)

Batch preparation and storage:

Foil blisters were filled and heat-sealed with fill weights targeting 1.0mg delivered dose:

- 100% Tacrolimus: 1 mg
- 50% Tacrolimus / 50% Trehalose: 2mg

Blisters were stored 'open' and 'wrapped in foil pouch' respectively at environmental conditions of 25°C/60% RH (standard) and 40°C/75% RH (accelerated).
Aerodynamic particle size was measured by inertial impaction (NGI) and assay of solutions by HPLC.

Results

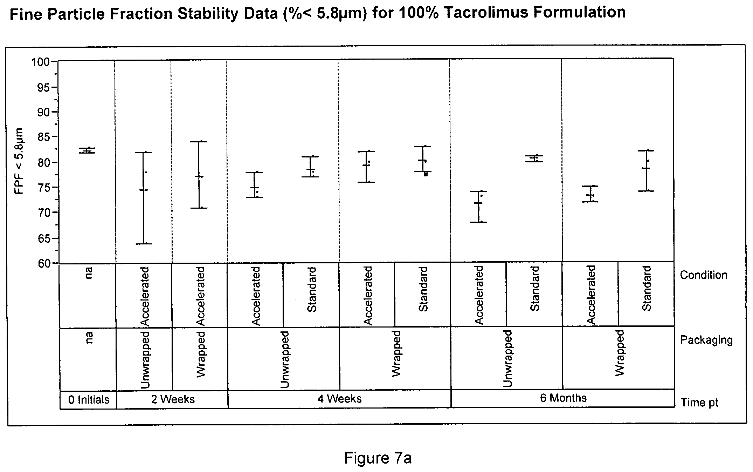

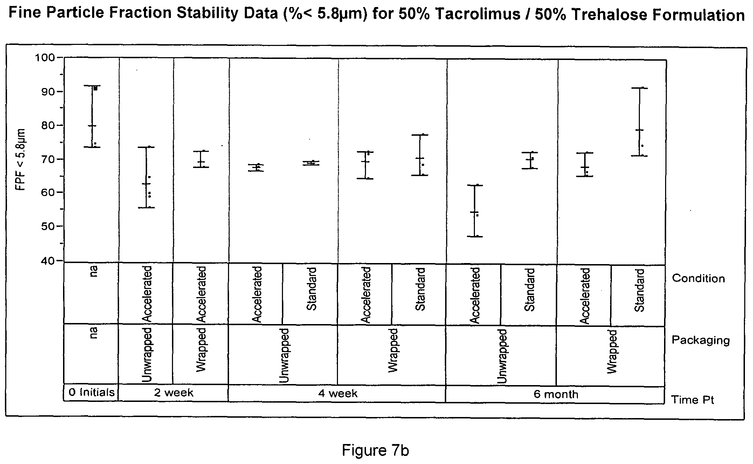

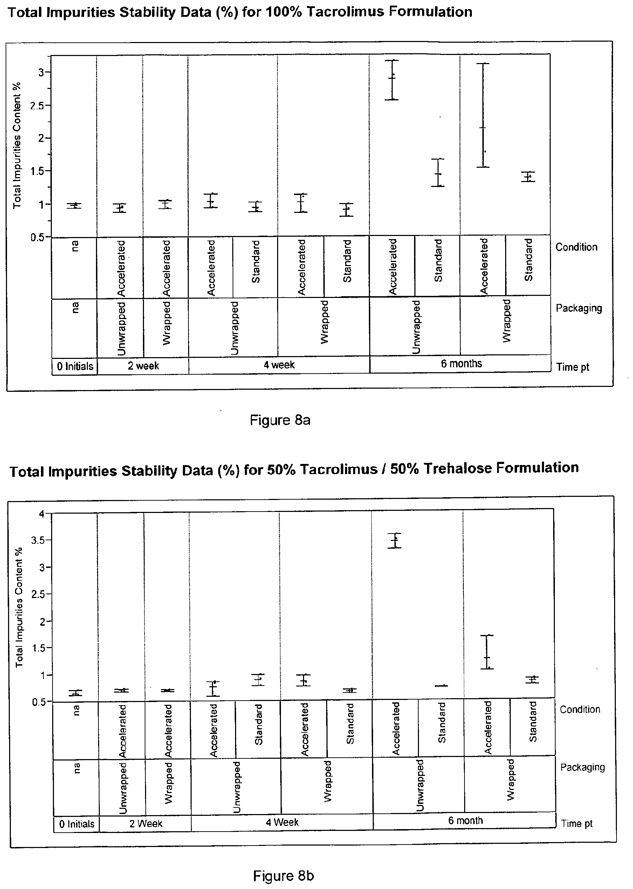

Physical stability (%FPF) was acceptable up to 4 weeks for both formulations at all conditions and acceptable up to 6 months for standard conditions. The results are shown in Figure 7.
The chemical stability (% total impurities by HPLC), was acceptable up to 4 weeks for both formulations at all conditions and acceptable up to 6 months for ambient conditions. The results are shown in Figure 8.
The data support a shelf life of up to 6 months at standard conditions.

## Example 10

Formulations were prepared with constant ratios of tacrolimus and trehalose levels where the ammonium bicarbonate loading was varied. The results are shown in Table 8.

Table 8

| | 50% Tacrolimus + 50% Trehalose | | | | |
|---|---|---|---|---|---|
| Ammonium Bicarbonate | **0%** | **20%** | **60%** | **80%** | **150%** |
| Mean Tacrolimus delivered dose ($\mu$g)[1] | 753 | 853 | 939 | 983 | 976 |
| | 573 | 718 | 755 | 772 | 816 |
| Mean FPF <5.8$\mu$m (%) | | 85 | 80 | 79 | 84 |
| MMAD ($\mu$m) | 76 2.5 | 2.1 | 2.6 | 2.8 | 2.2 |
| Particle size[3]: | | | | | |
| D10 ($\mu$m) | 1.42 | 1.37 | 1.19 | ND | 1.49 |
| D50 ($\mu$m) | 2.30 | 2.24 | 2.18 | ND | 2.61 |
| D90 ($\mu$m) | 3.76 | 3.62 | 3.75 | ND | 4.26 |
| Untapped density (g/cm$^3$) | 0.16 | 0.07 | 0.05 | 0.04 | 0.08 |
| Tapped density (g/cm$^3$) | 0.23 | 0.09 | 0.08 | 0.07 | 0.10 |
| Carr's Index (%) | 33 | 24 | 31 | 36 | 21 |

(continued)

| | 100% Tacrolimus | | | | |
|---|---|---|---|---|---|
| Ammonium Bicarbonate | 0% | 20% | 60% | 100% | 150% |
| Mean Tacrolimus delivered dose ($\mu$g) [1] | 672 | 769 | 1016 | 1820 | 883 |
| Mean FPM <5.8$\mu$m ($\mu$g) | 610 | 675 | 802 | 1481 | 734 |
| Mean FPF <5.8$\mu$m (%) | 91 | 88 | 81 | 82 | 83 |
| MMAD ($\mu$m) | 2.2 | 2.1 | 2.1 | 1.8 | 2.1 |
| Particle size: | | | | | |
| D10 ($\mu$m) | 1.62 | 1.45 | 1.24 | ND | 1.09 |
| D50 ($\mu$m) | 2.70 | 2.35 | 1.97 | ND | 1.80 |
| D90 ($\mu$m) | 4.11 | 3.94 | 3.36 | ND | 2.89 |
| Untapped density (g/cm$^3$) | 0.15 | 0.15 | 0.24 | ND | ND |
| Tapped density (g/cm$^3$) | 0.22 | 0.19 | 0.30 | ND | ND |
| Carr's Index (%) | 32 | 22 | 20 | ND | ND |
| [1] ex-device calculated from NGI data<br>ND Not determined | | | | | |

From the data it can be seen that the presence of ammonium bicarbonate reduces the Carr's index and thereby improves the flow properties of the powder. Surprisingly, as the level of ammonium bicarbonate is increased the Carr's index increases or is not measurable due to the highly cohesive nature of the formulations.

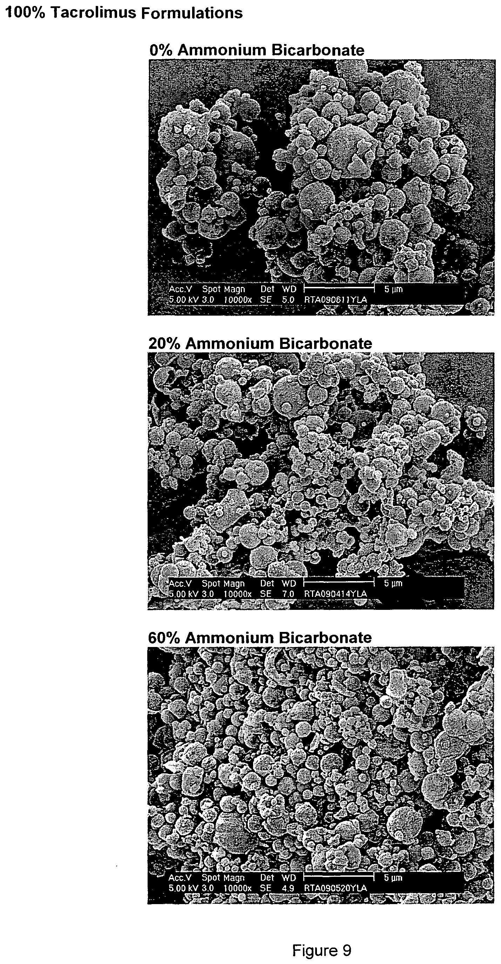

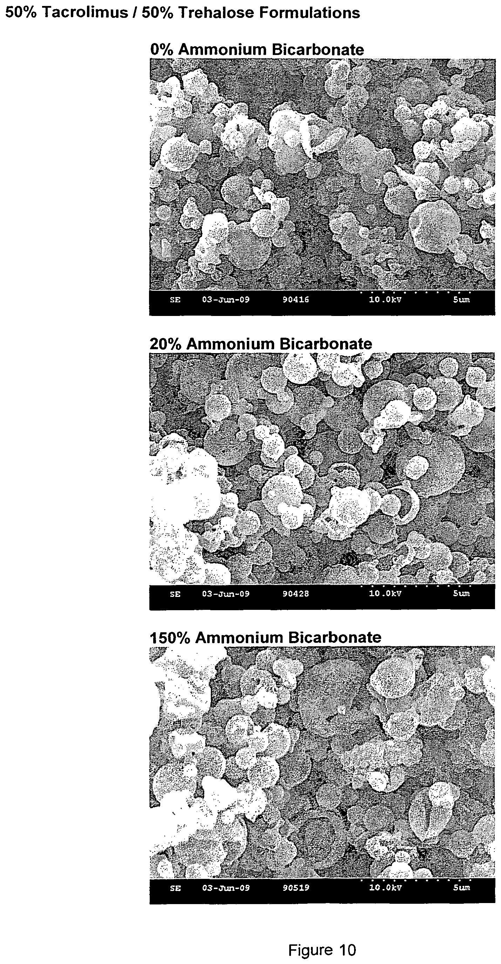

SEM images of the formulations manufactured with different amounts of ammonium bicarbonate are shown in Figures 9 and 10.

## Example 11

A formulation prepared from a spray dried ethanol/water solution containing Tacrolimus (50%), mannitol (50%) and ammonium bicarbonate (60%) has been compared with a formulation prepared from a spray dried ethanol/water solution containing Tacrolimus (50%), trehalose (50%) and ammonium bicarbonate (60%). This comparison is shown in Table 9.

Table 9

| | Mannitol | Trehalose |
|---|---|---|
| Mean Tacrolimus delivered dose ($\mu$g) [1] | 769 | 939 |
| Mean FPM <5.8$\mu$m ($\mu$g) | 564 | 755 |
| Mean FPF <5.8$\mu$m (%) | 73 | 80 |
| MMAD ($\mu$m) | 1.9 | 2.6 |
| Untapped density (g/cm$^3$) | 0.21 | 0.05 |
| Tapped density (g/cm$^3$) | 0.30 | 0.08 |
| Carr's Index (%) | 29 | 31 |

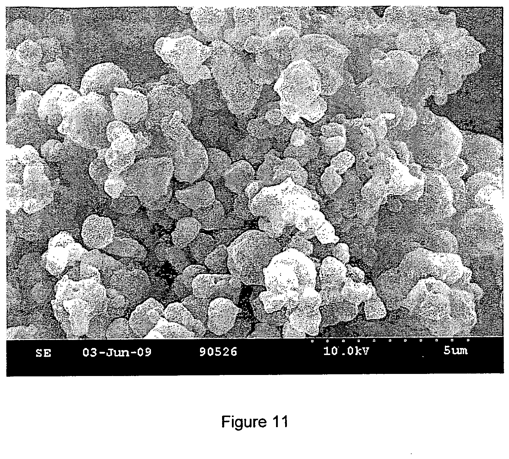

An SEM image of the formulation containing mannitol is shown in Figure 11.

DSC analysis of the formulation containing mannitol indicates that the mannitol is predominately crystalline.

## Example 12

Trehalose placebo batches were spray dried with different levels of ammonium bicarbonate. Physical characterisation data are presented in Table 10.

Table 10

| | | 100% Trehalose | | | | |
|---|---|---|---|---|---|---|
| Ammonium Bicarbonate | %w/w[1] | **0%** | **5%** | **5%** | **18%** | **18%** |
| | %w/v[2] | **0%** | **1%** | **1%** | **1%** | **4%** |
| Particle size[3]: | | | | | | |
| X10 ($\mu$m) | | 0.72 | 0.93 | 0.92 | 0.57 | 1.84 |
| X50 ($\mu$m) | | 1.69 | 3.18 | 2.92 | 1.68 | 5.53 |
| X90 ($\mu$m) | | 3.57 | 7.34 | 6.48 | 4.11 | 12.70 |
| Surface Area ($m^2/g$)[4] | | 2.605 | 1.752 | 1.822 | 3.488 | 6.744 |
| Untapped density ($g/cm^3$) | | 0.35 | 0.30 | 0.32 | 0.26 | ND |
| Tapped density ($g/cm^3$) | | 0.48 | 0.44 | 0.46 | 0.37 | ND |
| Carr's Index (%) | | 27 | 32 | 30 | 31 | ND |

[1] Based on the mass of trehalose in the feedstock solution
[2] basted on the volume of feedstock solution
[3] Median geometric particle size by laser diffraction
[4] Surface area by BET
ND Not determined

**Claims**

1. A process for preparing microparticles, which comprises the step of atomising a solution or dispersion comprising a saccharide and a blowing material in a carrier into a gas in order to obtain microparticles by evaporation of the carrier and removal, or decomposition and removal of the blowing material, wherein the carrier for the solution or dispersion is an aqueous carrier which does not comprise a liquid of greater volatility than water, wherein the microparticles have a tap density of equal to or less than 0.3 $g/cm^3$ and a median geometric diameter of less than, or equal to, 10 $\mu$m, and wherein the blowing material is selected from ammonium carbonate and ammonium bicarbonate or mixtures thereof.

2. The process according to Claim 1, wherein the microparticles have a mass median aerodynamic diameter of equal to or less than 10 $\mu$m.

3. The process according to Claim 2, wherein the microparticles have a mass median aerodynamic diameter of from 1 to 5 $\mu$m and preferably a median geometric diameter of less than 5 $\mu$m.

4. The process according to any one of Claims 1 to 3 wherein the saccharide is a monosaccharide, disaccharide, trisaccharide or mixture thereof.

5. The process according to Claim 4 wherein the saccharide is trehalose.

6. The process according to any one of Claims 1 to 5 wherein the microparticles comprise the saccharide in an amount of at least 50 wt.%, preferably 60, 70, 80 or 90 wt.%.

7. The process according to any one of Claims 1 to 6 wherein the solution or dispersion comprises a therapeutic agent, which is an enzyme, growth hormone, growth factor, insulin, antibodies, both monoclonal, polyclonal and fragments thereof, interferons, interleukins and cytokines.

8. The process according to Claim 7 wherein the therapeutic agent is an antibody or antibody fragment.

9. The process according to any one of Claims 1 to 8 wherein the solution or dispersion comprises a force control agent.

**10.** The process according to Claim 9 wherein the force control agent is leucine.

**11.** The process according to any one of Claims 1 to 10 wherein the microparticles have a bulk density of less than or equal to 0.2 g/cm$^3$.

## Patentansprüche

**1.** Vorgang zum Herstellen von Mikropartikeln, umfassend den Schritt des Zerstäubens einer Lösung oder Dispersion, die ein Saccharid und ein Treibmaterial in einem Träger umfasst, in ein Gas, um Mikropartikel durch Verdampfen des Trägers und Entfernen oder durch Zersetzen und Entfernen des Treibmaterials zu erhalten, wobei der Träger für die Lösung oder Dispersion ein wässriger Träger ist, der keine Flüssigkeit mit höherer Flüchtigkeit als Wasser umfasst, wobei die Mikropartikel eine Rütteldichte gleich oder kleiner als 0,3 g/cm$^3$ und einen mittleren geometrischen Durchmesser von kleiner oder gleich 10 $\mu$m aufweisen und wobei das Treibmaterial aus Ammoniumcarbonat und Ammoniumhydrogencarbonat oder Mischungen davon ausgewählt ist.

**2.** Vorgang nach Anspruch 1, wobei die Mikropartikel einen medianen massenbezogenen aerodynamischen Durchmesser von gleich oder kleiner als 10 $\mu$m aufweisen.

**3.** Vorgang nach Anspruch 2, wobei die Mikropartikel einen medianen massenbezogenen aerodynamischen Durchmesser von 1 bis 5 $\mu$m aufweisen und vorzugsweise einen mittleren geometrischen Durchmesser von weniger als 5 $\mu$m aufweisen.

**4.** Vorgang nach einem der Ansprüche 1 bis 3, wobei das Saccharid ein Monosaccharid, Disaccharid, Trisaccharid oder eine Mischung davon ist.

**5.** Vorgang nach Anspruch 4, wobei das Saccharid Trehalose ist.

**6.** Vorgang nach einem der Ansprüche 1 bis 5, wobei die Mikropartikel das Saccharid in einer Menge von mindestens 50 Gew.-%, vorzugsweise 60, 70, 80 oder 90 Gew.-% umfassen.

**7.** Vorgang nach einem der Ansprüche 1 bis 6, wobei die Lösung oder Dispersion ein therapeutisches Mittel umfasst, das ein Enzym, ein Wachstumshormon, ein Wachstumsfaktor, Insulin, sowohl monoklonale als auch polyklonale Antikörper sowie Fragmente davon, Interferone, Interleukine und Zytokine ist.

**8.** Vorgang nach Anspruch 7, wobei das therapeutische Mittel ein Antikörper oder ein Antikörperfragment ist.

**9.** Vorgang nach einem der Ansprüche 1 bis 8, wobei die Lösung oder Dispersion ein Kraftkontrollmittel (FCA, force control agent) umfasst.

**10.** Vorgang nach Anspruch 9, wobei das Kraftkontrollmittel Leucin ist.

**11.** Vorgang nach einem der Ansprüche 1 bis 10, wobei die Mikropartikel eine Schüttdichte von gleich oder kleiner als 0,2 g/cm$^3$ aufweisen.

## Revendications

**1.** Procédé de préparation de microparticules, qui comprend l'étape d'atomisation d'une solution ou dispersion comprenant un saccharide et un agent gonflant dans un vecteur dans un gaz afin d'obtenir des microparticules par évaporation du vecteur et élimination, ou décomposition et élimination, de l'agent gonflant, dans lequel le vecteur pour la solution ou dispersion est un vecteur aqueux qui ne comprend pas un liquide de volatilité supérieure à l'eau, dans lequel les microparticules ont une masse volumique après tassement égale ou inférieure à 0,3 g/cm$^3$ et un diamètre géométrique médian inférieur ou égal à 10 $\mu$m, et dans lequel le matériau gonflant est choisi parmi le carbonate d'ammonium et le bicarbonate d'ammonium ou des mélanges de ceux-ci.

**2.** Procédé selon la revendication 1, dans lequel les microparticules ont un diamètre aérodynamique massique médian égal ou inférieur à 10 $\mu$m.

**3.** Procédé selon la revendication 2, dans lequel les microparticules ont un diamètre aérodynamique massique médian compris entre 1 et 5 $\mu$m, et de préférence un diamètre géométrique médian inférieur à 5 $\mu$m.

**4.** Procédé selon l'une quelconque des revendications 1 à 3, dans lequel le saccharide est un monosaccharide, un disaccharide, un trisaccharide ou un mélange de ceux-ci.

**5.** Procédé selon la revendication 4, dans lequel le saccharide est le tréhalose.

**6.** Procédé selon l'une quelconque des revendications 1 à 5, dans lequel les microparticules comprennent le saccharide dans une quantité d'au moins 50 % en poids, de préférence 60, 70, 80 ou 90 % en poids.

**7.** Procédé selon l'une quelconque des revendications 1 à 6, dans lequel la solution ou dispersion comprend un agent thérapeutique, qui est une enzyme, une hormone de croissance, un facteur de croissance, une insuline, des anticorps, aussi bien monoclonaux que polyclonaux et des fragments de ceux-ci, des interférons, des interleukines et des cytokines.

**8.** Procédé selon la revendication 7, dans lequel l'agent thérapeutique est un anticorps ou un fragment d'anticorps.

**9.** Procédé selon l'une quelconque des revendications 1 à 8, dans lequel la solution ou dispersion comprend un agent de contrôle de la force.

**10.** Procédé selon la revendication 9, dans lequel l'agent de contrôle de la force est la leucine.

**11.** Procédé selon l'une quelconque des revendications 1 à 10, dans lequel les microparticules ont une masse volumique apparente inférieure ou égale à 0,2 g/cm$^3$.

SE    12-Dec-08      AKSA    WD 9.1mm 10.0kV         10um

Figure 1

**Comparison of ACI stage deposition of spray dried heparin : leucine and spray dried heparin with blowing agent (ammonium bicarbonate)**

Figure 2

**Comparison of ACI stage deposition of different spray dried heparin with different blowing agents (ammonium carbonate and ammonium bicarbonate)**

Figure 3

Low density

Figure 4

Standard heparin: leucine formulation

Figure 5

Figure 6

# Fine Particle Fraction Stability Data (%< 5.8µm) for 100% Tacrolimus Formulation

Figure 7a

EP 2 429 497 B1

Fine Particle Fraction Stability Data (%< 5.8μm) for 50% Tacrolimus / 50% Trehalose Formulation

Figure 7b

Total Impurities Stability Data (%) for 100% Tacrolimus Formulation

Figure 8a

Total Impurities Stability Data (%) for 50% Tacrolimus / 50% Trehalose Formulation

Figure 8b

## 100% Tacrolimus Formulations

### 0% Ammonium Bicarbonate

### 20% Ammonium Bicarbonate

### 60% Ammonium Bicarbonate

Figure 9

## 50% Tacrolimus / 50% Trehalose Formulations

### 0% Ammonium Bicarbonate

### 20% Ammonium Bicarbonate

### 150% Ammonium Bicarbonate

Figure 10

Figure 11

# EP 2 429 497 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9932083 A
- WO 03080028 A
- WO 2007086039 A
- WO 2004112702 A
- US 6565885 B
- WO 2005025540 A
- EP 1632208 A
- WO 2004041278 A
- WO 2005063242 A
- WO 9710806 A
- WO 9014826 A
- IN 200600953 A
- WO 2008127746 A
- US 6395300 B
- WO 2004030659 A
- US 5260301 A
- US 5196437 A
- US 5665727 A
- US 5089481 A
- WO 03068254 A
- EP 1511466 A
- WO 9218164 A
- WO 9615814 A
- WO 9817257 A
- US 2007022830 W
- US 6257233 B
- EP 2010050790 W

**Non-patent literature cited in the description**

- **CHOUGULE et al.** *Int. J. Nanomedicine,* 2007, vol. 2 (4), 675-88
- **SISWATA et al.** *European Journal of Pharmaceutics and Biopharmaceutics,* August 2008, vol. 69 (3), 1057-1066
- **R.L. CARR.** *Chem Eng.,* 1965, vol. 72, 163-168
- *Journal of Pharmaceutical Sciences,* 2009, vol. 98 (8), 2770-2783